# EUROPEAN PATENT APPLICATION

(11) **EP 4 566 596 A1**
(43) Date of publication of application: **11.06.2025**
(21) Application number: 23850036.7
(22) Date of filing: 31.07.2023
(51) Int. Cl.: A61K 31/167, A61K 31/275, A61K 31/5375, A61K 51/04, A61P 31/20, C07C 237/42, C07C 255/60, C12N 15/11

(54) **ANTI-HBV AGENT**

(30) Priority: 01.08.2022 JP 2022122769
(71) Applicant: RIKEN, Wako-shi, Saitama 351-0198 (JP)
(72) Inventor: FURUTANI Yutaka, Wako-shi, Saitama 351-0198 (JP); NIWA Takashi, Wako-shi, Saitama 351-0198 (JP); TSUDA Junpei, Wako-shi, Saitama 351-0198 (JP); OGAWA Kenji, Wako-shi, Saitama 351-0198 (JP); IWASAKI Wakana, Wako-shi, Saitama 351-0198 (JP); HOSOYA Takamitsu, Wako-shi, Saitama 351-0198 (JP); SHIROUZU Mikako, Wako-shi, Saitama 351-0198 (JP); KOBAYASHI Kaoru, Kiyose-shi, Tokyo 204-8588 (JP); KANAYAMA Yosuke, Wako-shi, Saitama 351-0198 (JP); NAKAOKA Takayoshi, Wako-shi, Saitama 351-0198 (JP); WATANABE Yasuyoshi, Wako-shi, Saitama 351-0198 (JP); MATSUURA Tomokazu, Hiratsuka-shi, Kanagawa 254-0034 (JP)
(74) Representative: Dehns
(86) International application number: PCT/JP2023/027926
(87) International publication number: WO 2024/029481

(57) **Abstract**

An anti-HBV agent containing a compound of Formula (I) or a salt thereof, where R¹ is a phenyl group substituted with a group represented by -CN or R³-NH-CO- and an alkyl group having 1 to 6 carbon atoms, where R³ is an alkyl group having 1 to 10 carbon atoms or a group represented by the formula R⁴-(CH₂)ₙ₁-, where R⁴ is CH₂=CH-, a phenyl group, a cyclohexyl group, or a morpholino group, and n1 is an integer of 1 to 5; and R² is a phenyl group substituted with a halogen.

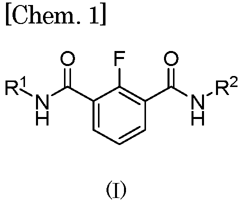

## Description

### Technical Field

The present invention relates to a novel anti-HBV agent.

### Background Art

Hepatitis B is caused by infection with the hepatitis B virus (HBV) through blood or body fluids, and it is estimated that about 10% of infected patients develop chronic hepatitis B. Chronic hepatitis B is a life-threatening disease that can progress to cirrhosis, liver failure, or hepatocellular carcinoma. Currently, there is no curative treatment method, and only palliative treatment methods exist. These treatment methods prevent the production of new HBV and the spread of infection, while waiting for infected cells to decrease or die through immunity and metabolism, or for HBV to become inactivated.

Currently, chronic hepatitis B is treated with interferon preparations that remove infected cells through innate immunity and nucleotide/nucleoside analog preparations that inhibit the reverse transcription of HBV-DNA. In particular, nucleotide/nucleoside analogue preparations exhibit high therapeutic effects by suppressing the proliferation of HBV-DNA, but it can be extremely difficult to determine when to stop administering the medication. HBV is a DNA virus that exists in the nuclei of hepatocytes in the form of cccDNA (covalently closed circular DNA). When the virus infects a hepatocyte, it enters the nucleus and produces pregenomic RNA. This RNA is used to synthesize HBV-DNA and various antigenic proteins, and therefore serves as a template to form new viral particles and supports proliferation (see the scheme below).

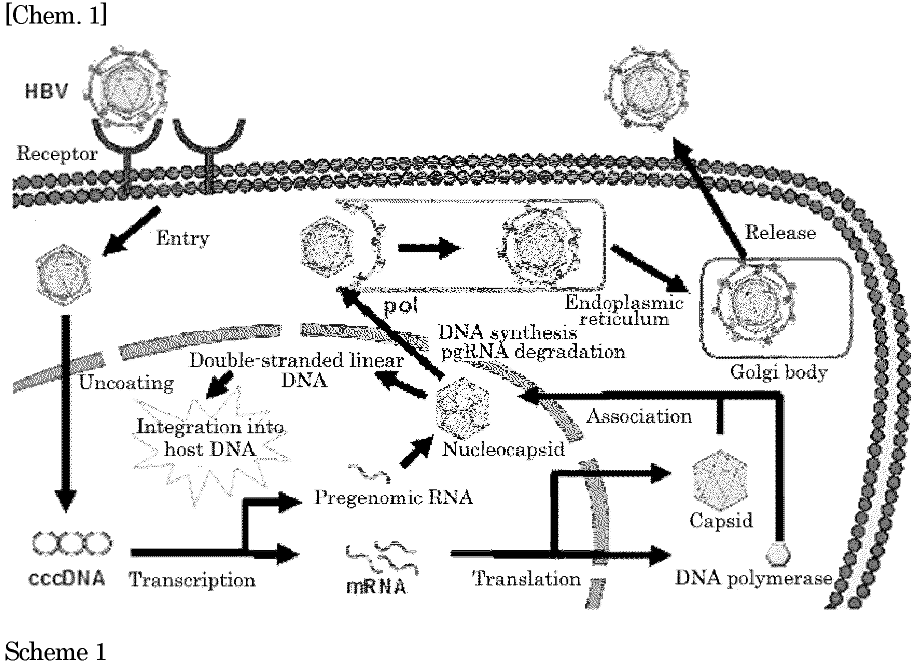

Currently, nucleotide/nucleoside analog preparations, exemplified by Entecavir (ETV), are widely used to treat chronic hepatitis B. These drugs have been somewhat effective in temporarily reducing the virus and delaying the onset of cirrhosis and hepatocellular carcinoma. However, persistently infected HBV is not completely eliminated from the body, and the emergence of drug-resistant viruses due to long-term administration has also been noted. Thus, there is a need to develop a novel drug with a different mechanism of action from nucleotide/nucleoside analogues.

The use of a capsid protein inhibitor as a therapeutic agent for viral infections caused by HBV or other viruses has been reported in recent years (Patent Document 1). In this invention, the 149 amino acid residues at the N terminal that constitute the core protein dimerization motif and the assembly domain do not have a sequence homologous to human proteins, and therefore, the capsid protein inhibitor is considered as a new target for anti-HBV drug development.

In addition, a therapeutic agent for treating a disease associated with pregenomic RNA capsid formation has also been reported (Patent Document 2). In this invention, it is also specified that the disease associated with pregenomic RNA capsid formation is hepatitis B virus infection.

Furthermore, the synthesis and evaluation of N-phenyl-3-sulfamoylbenzamide derivatives as HBV capsid allosteric modulators have also been reported (Non-Patent Document 1).

### Prior Art Documents

### Patent Documents

[Patent Document 1] WO 2019/20070
[Patent Document 2] WO 2014/106019

### Non-Patent Document

[Non-Patent Document 1] Vandyck, Koen et. al., Journal of Medicinal Chemistry, 2018, Vol. 61 (14), p. 6247-6260

### Disclosure of Invention

### Problem to be Solved by the Invention

HBV core protein (HBc) is an important protein that is a major component of the nucleocapsid of viral particles and plays an essential role in genome replication. The inventors of the present invention focus on the possibility of HBc serving as a potential drug target and aim to provide a novel anti-HBV agent that inhibits the formation of normal nucleocapsids.

### Means for Solving Problem

The inventors of the present invention have found a compound that inhibits normal nucleocapsid formation. They have also discovered that various derivatives based on this compound have the activity to inhibit HBV replication. Moreover, they have found that, out of such derivatives, certain compounds have the effect of suppressing not only HBV DNA but also cccDNA, HBsAg, and HBeAg. A problem with hepatitis B is that, even with treatment using a nucleotide/nucleoside analog preparation, cccDNA cannot be eliminated and may reactivate. Compounds that have the effect of suppressing cccDNA can suppress the reactivation and are therefore preferable. Additionally, while interferon preparations act on host cells and exert antiviral activity, certain compounds act directly on HBc of HBV, which is expected to reduce side effects. Based on these findings, the inventors of the present invention have developed a novel anti-HBV agent that inhibits the formation of normal nucleocapsids, thus achieving the present invention.

The present invention provides an anti-HBV agent containing a compound of Formula (I) or a salt thereof.

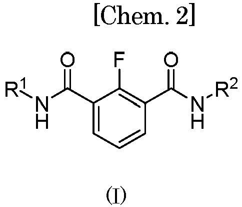

In the formula above,
R¹ is a phenyl group substituted with a group represented by -CN or R³-NH-CO- and an alkyl group having 1 to 6 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 5; and
R² is a phenyl group substituted with a halogen.

### Effects of the Invention

The present invention provides a novel anti-HBV agent.

### Brief Description of Drawings

[FIG. 1] FIG. 1 shows the results of mouse PET imaging.
[FIG. 2] FIG. 2 shows the results regarding the effect of suppressing HBsAg, HBeAg, and pgRNA.
[FIG. 3] FIG. 3 shows the results regarding the effect of suppressing cccDNA and pgRNA.
[FIG. 4] FIG. 4 shows the results of an experiment for measuring the anti-HBV activity of compound CBT-209 using hepatitis B virus-infected human hepatocyte chimeric mice.
[FIG. 5] FIG. 5 shows the results of analysis of the mechanism of capsid formation inhibition by electron microscopy.

### Description of the Invention

The present invention provides an anti-HBV agent containing the compound of Formula (I) or salt thereof.

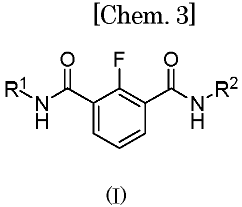

In the formula above,
R¹ is a phenyl group substituted with a group represented by -CN or R³-NH-CO- and an alkyl group having 1 to 6 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 5; and
R² is a phenyl group substituted with a halogen.

Also, the present invention provides a compound of Formula (I) or a salt thereof.

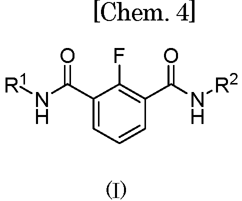

In the formula above,
R¹ is a phenyl group substituted with a group represented by -CN or R³-NH-CO- and an alkyl group having 1 to 6 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 5; and
R² is a phenyl group substituted with a halogen.

Also, the present invention provides a compound of Formula (II) or a salt thereof.

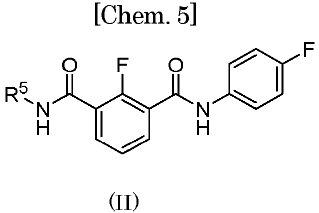

In the formula above,
R⁵ is a phenyl group substituted with a group represented by R⁶-NH-CO- and an alkyl group having 1 to 6 carbon atoms,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 1 to 5.

Also, the present invention provides a compound of Formula (III) or a salt thereof.

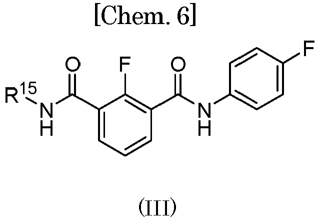

In the formula above,
R¹⁵ is a phenyl group substituted with a group represented by R¹⁶-NH-CO- and an alkyl group having 1 to 6 carbon atoms,
where R¹⁶ is an alkyl group having 1 to 10 carbon atoms or a group represented by the formula R¹⁷-(CH₂)ₙ₃-,
where R¹⁷ is CH₂=CH-, a phenyl group, a cyclohexyl group, or a morpholino group, and
n3 is an integer of 1 to 5,
the compound of Formula (III) being labeled with ¹¹C, or labeled with ¹⁸F when the compound contains a fluorine atom.

Also, the present invention provides an HBV capsid allosteric modulators containing the compound of Formula (I) or salt thereof or the compound of Formula (II) or salt thereof.

Also, the present invention provides a PET probe for imaging that targets a hepatitis B antigen protein, the PET probe containing the compound of Formula (III) or salt thereof.

### Definitions

In the present invention, the "halogen" may be fluorine, chlorine, bromine, or iodine, preferably fluorine, chlorine, or bromine, and more preferably fluorine.

In the present invention, the "alkyl having 1 to 6 carbon atoms" may be methyl, ethyl, n-propyl, isopropyl, 2-methylpropyl, n-butyl, t-butyl, pentyl, hexyl, or the like. The "alkyl having 1 to 6 carbon atoms" may preferably be an alkyl having 1 to 5 carbon atoms, more preferably an alkyl having 1 to 4 carbon atoms, and even more preferably an alkyl having 1 to 3 carbon atoms.

In the present invention, the "alkyl having 1 to 10 carbon atoms" may be methyl, ethyl, n-propyl, isopropyl, 2-methylpropyl, n-butyl, t-butyl, pentyl, hexyl, heptyl, octyl, nonyl, decanyl, or the like. The "alkyl having 1 to 10 carbon atoms" may preferably be an alkyl having 2 to 10 carbon atoms, and more preferably an alkyl having 1 to 8 carbon atoms.

In the present invention, the "alkyl having 1 to 3 carbon atoms" may be methyl, ethyl, n-propyl, isopropyl, or the like. The "alkyl having 1 to 3 carbon atoms" may preferably be an alkyl having 1 to 2 carbon atoms, and more preferably an alkyl having 2 carbon atoms.

In the present invention, the salt may be a commonly used salt that is nontoxic and pharmaceutically acceptable, and examples thereof include: salts with alkali metals such as sodium and potassium; salts with alkaline earth metals such as calcium and magnesium; salts with inorganic bases such as ammonium; salts with organic amines such as triethylamine, pyridine, picoline, ethanolamine, triethanolamine, dicyclohexylamine, and N,N'-dibenzylethyleneamine; salts with inorganic acids such as hydrochloric acid, hydrobromic acid, sulfuric acid, and phosphoric acid; salts with organic carboxylic acids such as formic acid, acetic acid, trifluoroacetic acid, maleic acid, and tartaric acid; salts with sulfonic acids such as methanesulfonic acid, benzenesulfonic acid, and p-toluenesulfonic acid; and salts with bases or acids such as arginine, aspartic acid, glutamic acid, and other basic or acidic amino acids.

### Anti-HBV Agent

Compounds of Formula (I) have higher anti-HBV activity than conventional compounds. In particular, the anti-HBV activity in HBV-infected human hepatocyte chimeric mice was higher than that of conventional compounds. Moreover, compounds of Formula (I) have the effect of suppressing cccDNA and the effect of being able to suppress the reactivation of hepatitis B.

In the anti-HBV agent of the present invention, the compound of Formula (I) is preferably a compound of Formula (I),
where
R¹ is a phenyl group substituted with a group represented by -CN or R³-NH-CO- and an alkyl group having 1 to 3 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 3; and
R² is a phenyl group substituted with a halogen.

In the anti-HBV agent of the present invention, the compound of Formula (I) is more preferably a compound of Formula (I),
where
R¹ is a phenyl group substituted with a group represented by -CN or R³-NH-CO- and an alkyl group having 1 carbon atom,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 2; and
R² is a phenyl group substituted with fluorine.

In the anti-HBV agent of the present invention, compounds listed in Table 1 below are preferable as the compound of Formula (I).

**[Table 1]**

| 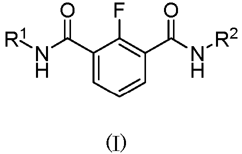 | | |
|---|---|---|
| | R¹ | R² |
| CBT-209 | 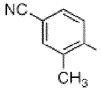 | 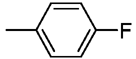 |
| CBT-289 | 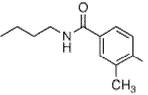 | 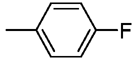 |
| CBT-383 | 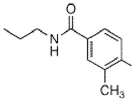 | 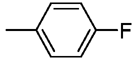 |
| CBT-384 | 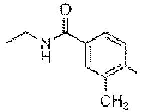 | 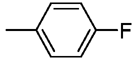 |
| CBT-385 | 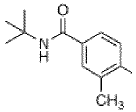 | 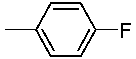 |
| CBT-387 | 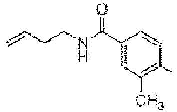 | 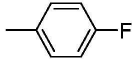 |
| CBT-388 | 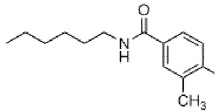 | 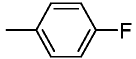 |
| CBT-389 | 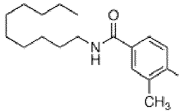 | 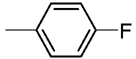 |
| CBT-390 | 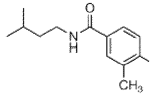 | 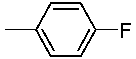 |
| CBT-391 | 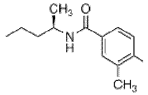 | 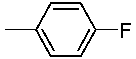 |
| CBT-400 | 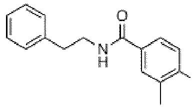 | 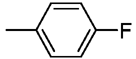 |
| CBT-401 | 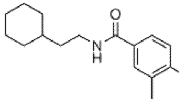 | 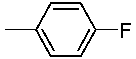 |
| CBT-402 | 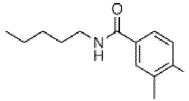 | 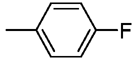 |
| CBT-403 | 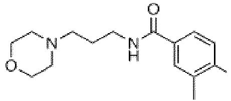 | 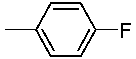 |
| CBT-404 | 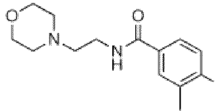 | 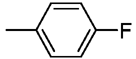 |

Also, in the anti-HBV agent of the present invention, the compound of Formula (I) is preferably a compound of Formula (II) below.

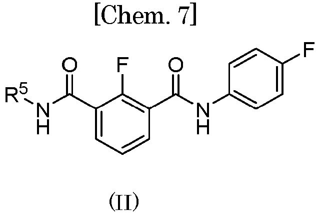

In the formula above,
R⁵ is a phenyl group substituted with a group represented by R⁶-NH-CO- and an alkyl group having 1 to 6 carbon atoms,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 1 to 5.

In the anti-HBV agent of the present invention, the compound of Formula (II) is preferably a compound of Formula (II),
where
R⁵ is a phenyl group substituted with a group represented by R⁶-NH-CO- and an alkyl group having 1 to 3 carbon atoms,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 1 to 3.

In the anti-HBV agent of the present invention, the compound of Formula (II) is more preferably a compound of Formula (II),
where
R⁵ is a phenyl group substituted with a group represented by R⁶-NH-CO- and an alkyl group having 1 carbon atom,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 2.

In the anti-HBV agent of the present invention, compounds listed in Table 2 below are preferable as the compound of Formula (II).

**[Table 2]**

| 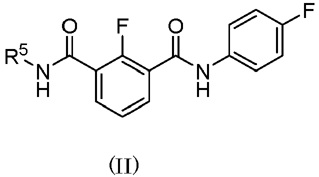 | | | |
|---|---|---|---|
| | R⁵ | | R⁵ |
| CBT-289 | 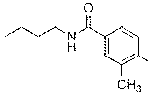 | CBT-390 | 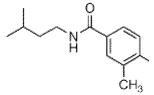 |
| CBT-383 | 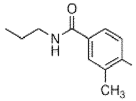 | CBT-391 | 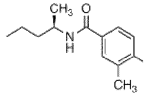 |
| CBT-384 | 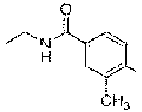 | CBT-400 | 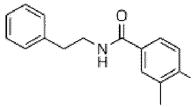 |
| CBT-385 | 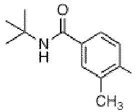 | CBT-401 | 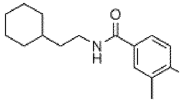 |
| CBT-387 | 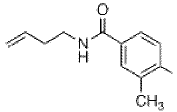 | CBT-402 | 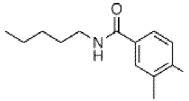 |
| CBT-388 | 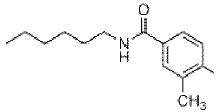 | CBT-403 | 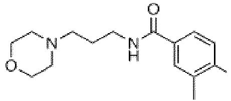 |
| CBT-389 | 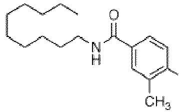 | CBT-404 | 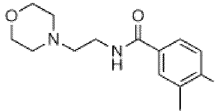 |

### Compounds

The present invention also provides a compound of Formula (I) or a salt thereof.

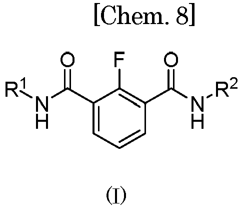

In the formula above,
R¹ is a phenyl group substituted with a group represented by -CN or R³-NH-CO- and an alkyl group having 1 to 6 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 5; and
R² is a phenyl group substituted with a halogen.

The compound of Formula (I) may be, for example, the compounds exemplified in Table 1 above.

The compound of Formula (I) is preferably a compound of Formula (I),
where
R¹ is a phenyl group substituted with a group represented by -CN or R³-NH-CO- and an alkyl group having 1 to 3 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 3; and
R² is a phenyl group substituted with a halogen.

The compound of Formula (I) is more preferably a compound of Formula (1),
where
R¹ is a phenyl group substituted with a group represented by -CN or R³-NH-CO- and an alkyl group having 1 carbon atom,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 2; and
R² is a phenyl group substituted with fluorine.

The present invention also provides a compound of Formula (II) or a salt thereof.

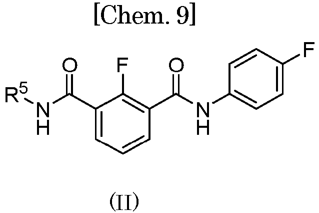

In the formula above,
R⁵ is a phenyl group substituted with a group represented by R⁶-NH-CO- and an alkyl group having 1 to 6 carbon atoms,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 1 to 5.

The compound of Formula (II) may be, for example, the compounds exemplified in Table 2 above.

The compound of Formula (II) is preferably a compound of Formula (I),
where
R⁵ is a phenyl group substituted with a group represented by R⁶-NH-CO- and an alkyl group having 1 to 3 carbon atoms,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 1 to 3.

The compound of Formula (II) is more preferably a compound of Formula (I),
where
R⁵ is a phenyl group substituted with a group represented by R⁶-NH-CO- and an alkyl group having 1 carbon atom,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 2.

### Compound Production Method

A method for producing the compound of Formula (I) of the present invention is as follows.

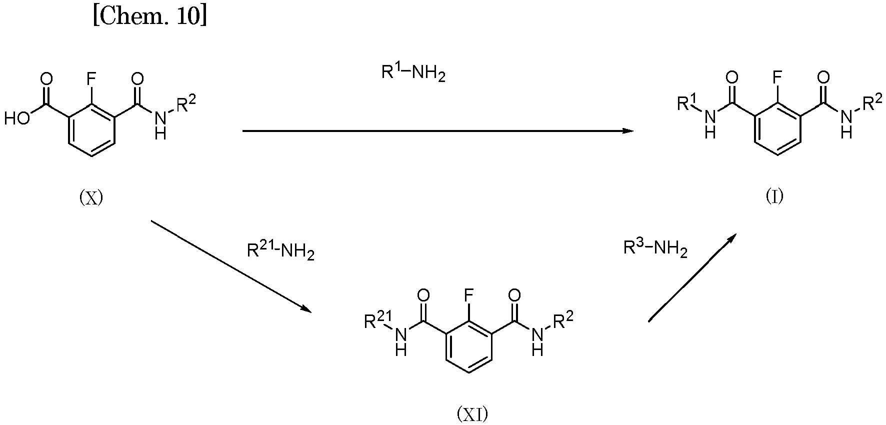

In the formulae above,
R¹ is a phenyl group substituted with a group represented by -CN or R³-NH-CO- and an alkyl group having 1 to 6 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 5; and
R² is a phenyl group substituted with a halogen.
R²¹ is a phenyl group substituted with a group represented by R³-NH-CO- and an alkyl group having 1 to 6 carbon atoms.

The compound of Formula (I) can be obtained by, for example, condensing a compound of Formula (X) with R¹-NH₂. Alternatively, the compound of Formula (I) can be obtained by condensing a compound of Formula (X) with R¹¹-NH₂ to obtain a compound of Formula (XI), and then condensing this compound with R³-NH₂.

The condensation step(s) can be performed using a conventionally known condensing agent. Known examples of the condensing agent include carbodiimide-, imidazole-, triazine-, phosphonium-, uronium-, and halonium-based condensing agents. Examples of the carbodiimide-based condensing agents include 1-[3-(dimethylamino)propyl]-3-ethyl carbodiimide (EDCI), 1-ethyl-3-(3-dimethylaminopropyl)carbodiimide hydrochloride (EDCI•HCl), N,N'-dicyclohexylcarbodiimide (DCC), and N,N'-diisopropylcarbodiimide (DIC). Examples of the imidazole-based condensing agents include N,N'-carbonyldiimidazole (CDI) and 1,1'-carbonyldi(1,2,4-triazole) (CDT). Examples of the triazine-based condensing agents include 4-(4,6-dimethoxy-1,3,5-triazin-2-yl)-4-methylmorpholinium chloride n-hydrate (DMT-MM). Examples of the phosphonium-based condensing agents include 1H-benzotriazol-1-yloxytris(dimethylamino)phosphonium hexafluorophosphate (BOP) and 1H-benzotriazol-1-yloxytripyrrolidinophosphonium hexafluorophosphate (PyBOP). Examples of the uronium-based condensing agents include {{[ (1-cyano-2-ethoxy-2-oxoethylidene)amino]oxy}-4-morpholinomethylene}dimethylammonium hexafluorophosphate (COMU). Examples of the halonium-based condensing agents include 2-chloro-1,3-dimethylimidazolinium hexafluorophosphate (CIP).

The compound represented by Formula (X) may be produced according to known literature, such as WO 2020/255425 or Japanese Patent No. 7072213, for example, or may be commercially available.

The present invention also provides a compound of Formula (III) or a salt thereof.

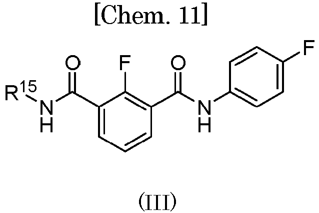

In the formula above,
R¹⁵ is a phenyl group substituted with a group represented by R¹⁶-NH-CO- and an alkyl group having 1 to 6 carbon atoms,
where R¹⁶ is an alkyl group having 1 to 10 carbon atoms or a group represented by the formula R¹⁷-(CH₂)ₙ₃-,
where R¹⁷ is CH₂=CH-, a phenyl group, a cyclohexyl group, or a morpholino group, and
n3 is an integer of 1 to 5.
The compound of Formula (III) is labeled with ¹¹C, or labeled with ¹⁸F when the compound contains a fluorine atom.

The compound of Formula (III) is preferably a compound of Formula (III),
where
R¹⁵ is a phenyl group substituted with a group represented by R¹⁶-NH-CO- and an alkyl group having 1 to 3 carbon atoms,
where R¹⁶ is an alkyl group having 1 to 10 carbon atoms or a group represented by the formula R¹⁷-(CH₂)ₙ₃-,
where R¹⁷ is CH₂=CH-, a phenyl group, a cyclohexyl group, or a morpholino group, and
n3 is an integer of 1 to 3.

The compound of Formula (III) is more preferably a compound of Formula (III),
where
R¹⁵ is a phenyl group substituted with a group represented by R¹⁶-NH-CO- and an alkyl group having 1 carbon atom,
where R¹⁶ is an alkyl group having 1 to 10 carbon atoms or a group represented by the formula R¹⁷-(CH₂)ₙ₃-,
where R¹⁷ is CH₂=CH-, a phenyl group, a cyclohexyl group, or a morpholino group, and
n3 is an integer of 2.

In the present invention, the phrase "The compound of Formula (III) is labeled with ¹¹C, or labeled with ¹⁸F when the compound contains a fluorine atom." means either (a) or (b) below. 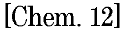 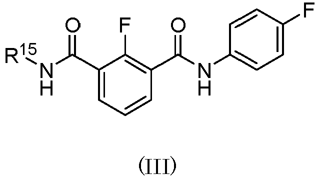
(a) Any one of the carbon atoms in Formula (III) above is labeled with ¹¹C.
(b) Fluorine (F) in Formula (III) above is labeled with ¹⁸F.

In the present invention, an example of the case where "The compound of Formula (III) is labeled with ¹¹C, or labeled with ¹⁸F when the compound contains a fluorine atom." is a compound shown below.

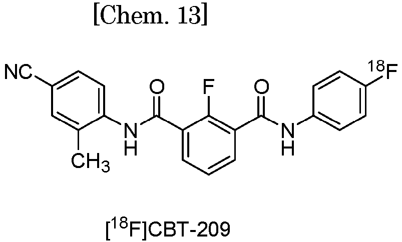

A compound that is "labeled with ¹¹C" of the present invention can be produced by combining the above-described compound production method of the present invention with conventionally known rapid ¹¹C-methylation (Chem. Rec. 2014, 14, 516-541.). A compound that is "labeled with ¹⁸F when the compound contains a fluorine atom" of the present invention can be produced by combining the above-described compound production method of the present invention with a conventionally known ¹⁸F-labeling method (Org. Lett. 2015, 17, 5780-5783; Chem. Commun. 2016, 52, 8361-8364).

The present invention also provides a PET probe for imaging that targets a hepatitis B antigen protein, the PET probe containing a compound of Formula (III) or a salt thereof.

The PET probe for imaging of the present invention can be formulated into an appropriate dosage form, such as an injectable form, using a conventionally known method. An injectable, for example, an injectable for intravenous administration can be formulated by adding a vehicle, a pH regulator, a buffer, a suspending agent, a solubilizing agent, an antioxidant, a preservative, and the like to a compound represented by the general formula (III) or a salt thereof, as necessary, and then treating the mixture using a conventional method.

### Abbreviation

The following abbreviation may be used in this specification.
THF: Tetrahydrofuran

### Production Example 1

### Synthesis of Compound of Formula (S2)

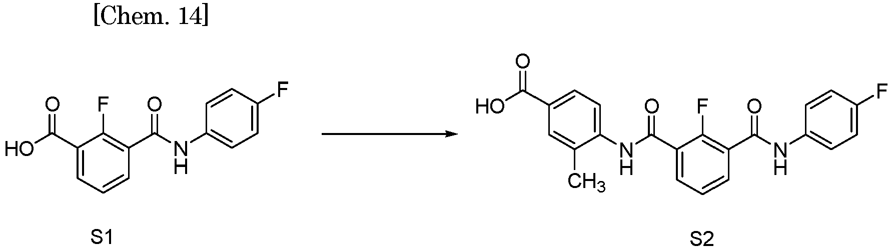

A compound of Formula (S1) was produced according to a method for producing 2-fluoro-3-((4-fluorophenyl)carbamoyl)benzoic acid (4) disclosed in JP 2019-112326A.

To a THF solution (25 mL) containing the compound of Formula (S1) (1.39 g, 5.0 mmol, 1 equivalent) was added thionyl chloride (3.65 mL, 7.5 mmol, 20 equivalents), and the resulting mixture was heated to 80°C and stirred for 2 hours. The mixture was then cooled to room temperature, and the solvent was removed from the mixture under reduced pressure. THF (25 mL), 3-methyl-4-aminobenzoic acid (0.89 mL, 5.0 mmol, 2.0 equivalents), and N,N-diisopropylethylamine (0.89 mL, 5.0 mmol, 1.0 equivalent) were added to the resulting residue, and the mixture was stirred at room temperature for 19 hours. A hydrochloric acid solution (1 mol/L, 20 mL) was added to the mixture, followed by stirring, and the resulting mixture was then extracted three times with ethyl acetate. Combined organic phases were washed with brine and dehydrated with anhydrous sodium sulfate. After the solvent was distilled off from the organic phases under reduced pressure, acetone was added to the solid residue, which was then heated to 70°C and dissolved in acetone. After cooling, a small amount of hexane was added to the acetone solution to precipitate the solid. The precipitated solid was collected by filtration to obtain a compound of Formula (S2) (1.31 g, 3.20 mmol, 64.0% yield).

¹H NMR (DMSO-d₆) δ 2.34 (s, 3H), 7.22 (dd, J = 8.8, 8.8 Hz, 2H), 7.45 (dd, J = 7.2, 8.0 Hz, 1H), 7.72-7.89 (m, 7H), 10.13 (s, 1H), 10.67 (d, J = 3.6 Hz, 1H); ¹⁹F NMR (DMSO-ds) δ -116.6 (1F), -119.8 (1F);
HRMS (ESI⁻) m/z 409.0996 (calcd. for C₂₂H₁₅F₂N₂O₄, 405.1005, [M]⁻).

### General Method for Producing Compounds Described in Examples

To a tetrahydrofuran (THF) solution containing a carboxylic acid (compound of Formula (S1) or compound of Formula (S2), 1 equivalent) was added thionyl chloride (20 equivalents), and the resulting mixture was heated to 80°C and stirred for 2 hours. The mixture was then cooled to room temperature, and the solvent was removed from the mixture under reduced pressure. THF, an amine compound (2.0 equivalents), and N,N-diisopropylethylamine (1.0 equivalent) were added to the resulting residue, and the mixture was stirred at room temperature for 20 hours. A hydrochloric acid solution (1 mol/L) was added to the mixture, followed by stirring, and the resulting mixture was then extracted three times with ethyl acetate. Combined organic phases were washed with brine and dehydrated with anhydrous sodium sulfate. After the solvent was distilled off from the organic phases under reduced pressure, the solid residue was purified by recrystallization (using acetone and hexane) or silica gel column chromatography to obtain a CBT compound.

### Example 1

### Production of Compound CBT-209

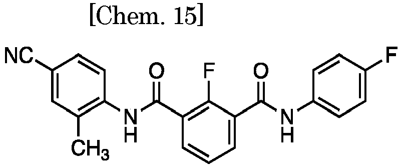

Production was performed according to the above-described general production method using the compound of Formula (S1) (2.77 g, 10.0 mmol) as the carboxylic acid and 2-methyl-4-cyanoaniline (1.98 g, 15 mmol, 1.5 equivalents) as the amine compound, and thus, a compound was produced (2.99 g, 7.6 mmol, 76.3% yield). Purification was performed by silica gel column chromatography (hexane/ethyl acetate = 9/1 to 1/1).

¹H NMR (acetone-d₆) δ 2.46 (s, 3H), 7.16 (dd, J = 8.8, 9.2 Hz, 2H), 7.49 (dd, J = 8.0, 7.6 Hz, 1H), 7.66-7.70 (m, 2H), 7.83-7.86 (m, 2H), 7.96 (dd, J = 7.6, 7.2 Hz, 1H), 8.04 (dd, J = 7.2, 7.6 Hz, 1H), 9.23 (s, 1H), 9.65 (s, 1H);
¹⁹F NMR (DMSO-d₆) δ -116.6 (1F), -119.6 (1F);
HRMS (ESI⁺) m/z 392.1208 (calcd. for C₂₂H₁₆F₂N₃O₂⁺, 3992.1205, [M+H]⁺).

### Example 2

### Production of Compound CBT-289

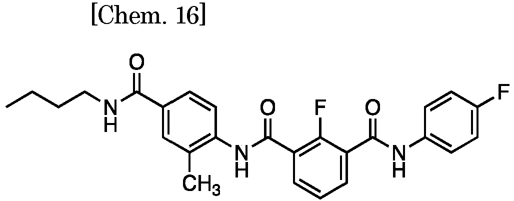

Production was performed according to the above-described general production method using the compound of Formula (S2) (205 mg, 0.5 mmol) as the carboxylic acid and n-butylamine (100 µL, 1.0 mmol, 2.0 equivalents) as the amine compound, and thus, a compound was produced (127 mg, 0.27 mmol, 54.3% yield). Purification was performed by recrystallization.

1H NMR (acetone-d₆) δ 0.93 (t, J = 7.2 Hz, 3H), 1.32-1.42 (m, 2H), 1.55-1.62 (m, 2H), 2.41 (s, 3H), 1.55-1.62 (td, J = 7.2, 7.2 Hz, 2H), 7.17 (dd, J = 7.2, 8.8 Hz, 2H), 7.48 (dd, J = 8.0, 7.6 Hz, 1H), 7.68 (br, 1H), 7.76-7.80 (m, 2H), 7.84-7.86 (m, 2H), 7.93 (dd, J = 6.4, 6.0 Hz, 1H), 8.01-8.03 (m, 2H), 9.16 (d, J = 4.4 Hz, 1H), 9.74 (s, 1H);
¹⁹F NMR (acetone-d₆) δ -116.5 (1F), -119.8 (1F);
HRMS (ESI⁺) m/z 466.1938 (calcd. for C₂₆H₂₆F₂N₃O₃⁺, 466.1937, [M+H]⁺).

### Example 3

### Production of Compound CBT-383

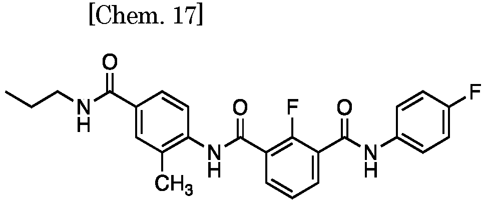

Production was performed according to the above-described general production method using the compound of Formula (S2) (41.1 mg, 0.100 mmol) as the carboxylic acid and n-propylamine (100 µL, 1.0 mmol, 2.0 equivalents) as the amine compound, and thus, a compound was produced (26.9 mg, 61.5 µmol, 61.5% yield). Purification was performed by recrystallization.

¹H NMR (DMSO-d₆) δ 0.89 (t, J = 7.6 Hz, 3H), 1.51-1.56 (m, 2H), 2.33 (s, 3H), 3.21 (dt, J = 6.4, 6.4 Hz, 2H), 7.22 (dd, J = 7.6, 8.8 Hz, 2H), 7.45 (dd, J = 7.2, 8.0 Hz, 1H), 7.61-7.87 (m, 7H), 8.42 (t, J = 5.2 Hz, 1H), 10.06 (s, 1H), 10.62 (s, 1H);
¹⁹F NMR (DMSO-d₆) δ -116.6 (1F), -118.3 (1F);
HRMS (ESI⁺) m/z 452.1780 (calcd. for C₂₅H₂₃F₂N₃O₃⁺, 452.1780, [M+H]⁺).

### Example 4

### Production of Compound CBT-384

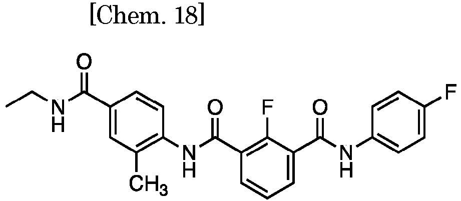

Production was performed according to the above-described general production method using the compound of Formula (S2) (41.1 mg, 0.100 mmol) as the carboxylic acid and an aqueous solution of ethylamine (10 mol/L, 20 µL, 0.20 mmol, 2.0 equivalents) as the amine compound, and thus, a compound was produced (37.2 mg, 85.0 µmol, 85.0%). Purification was performed by recrystallization.

¹H NMR (DMSO-d₆) δ 1.08 (t, J = 7.2 Hz, 3H), 2.28 (s, 3H), 3.24 (dq, J = 6.8, 6.8 Hz, 2H), 7.17 (dd, J = 7.6, 6.8 Hz, 2H), 7.41 (dd, J = 7.6, 8.0 Hz, 1H), 7.57-7.85 (m, 7H), 8.39 (t, J = 5.2 Hz, 1H), 10.02 (s, 1H), 10.58 (s, 1H);
¹⁹F NMR (DMSO-d₆) δ -116.6 (1F), -118.3 (1F);
HRMS (ESI⁺) m/z 438.1623 (calcd. for C₂₄H₂₁F₂N₃O₃⁺, 438.1624, [M+H]⁺).

### Example 5

### Production of Compound CBT-385

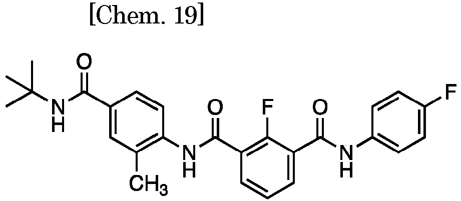

Production was performed according to the above-described general production method using the compound of Formula (S2) (41.1 mg, 0.100 mmol) as the carboxylic acid and 2-amino-2-methylpropane (21 µL, 0.2 mmol, 2.0 equivalents) as the amine compound, and thus, a compound was produced (36.0 mg, 77.2 pmol, 77.2% yield). Purification was performed by recrystallization.

¹H NMR (acetone-d₆) δ 1.45 (s, 9H), 2.39 (s, 3H), 7.08 (s, 1H), 7.16 (dd, J = 8.8, 8.8 Hz, 2H), 7.47 (dd, J = 8.0, 8.0 Hz, 1H), 7.69-7.74 (m, 2H), 7.83-7.87 (m, 2H), 7.91-8.02 (m, 3H), 9.10 (d, J = 2.0 Hz, 1H), 9.68 (s, 1H);
¹⁹F NMR (acetone-d₆) δ -116.8 (1F), -119.8 (1F);
HRMS (ESI⁺) m/z 466.1935 (calcd. for C₂₆H₂₆F₂N₃O₃⁺, 466.1937, [M+H]⁺).

### Example 6

### Production of Compound CBT-387

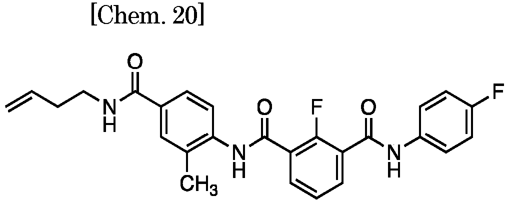

Production was performed according to the above-described general production method using the compound of Formula (S2) (41.1 mg, 0.100 mmol) as the carboxylic acid and 3-buten-1-amine hydrochloride (21 µL, 0.20 mmol, 2.0 equivalents) as the amine compound, and thus, a compound was produced (14.7 mg, 31.7 µmol, 31.7% yield). Purification was performed by recrystallization.

¹H NMR (acetone-d₆) δ 2.33-2.41 (m, 5H), 3.50 (td, J = 6.0, 7.2 Hz, 1H), 4.99-5.11 (m, 2H), 5.81-5.92 (m, 1H), 7.16 (dd, J = 9.2, 7.2 Hz, 2H), 7.47 (dd, J = 7.2, 8.0 Hz, 1H), 7.69 (br, 1H), 7.74-7.79 (m, 2H), 7.83-7.86 (m, 2H), 7.93 (dd, J = 7.2, 7.2 Hz, 1H), 8.02 (m, 2H), 9.12 (d, J = 6.0 Hz, 1H), 9.68 (s, 1H);
¹⁹F NMR (acetone-d₆) δ -116.7 (1F), -119.8 (1F);
HRMS (ESI⁺) m/z 464.1780 (calcd. for C₂₆H₂₄F₂N₃O₃⁺, 464.1780, [M+H]⁺).

### Example 7

### Production of Compound CBT-388

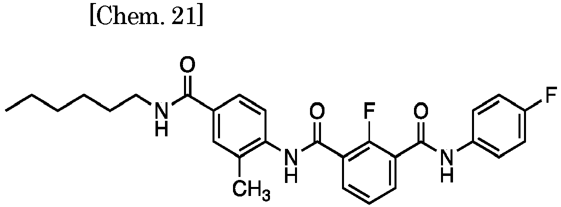

Production was performed according to the above-described general production method using the compound of Formula (S2) (41.1 mg, 0.100 mmol) as the carboxylic acid, n-hexylamine (26 µL, 0.20 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (21.0 mg, 42.5 µmol, 42.5% yield). Purification was performed by recrystallization.

¹H NMR (acetone-d₆) δ 0.89 (m, 3H), 1.32-1.40 (m, 6H), 1.59-1.62 (m, 2H), 2.41 (s, 3H), 3.38-3.39 (m, 2H), 7.17 (dd, J = 8.4, 6.8 Hz, 2H), 7.48 (dd, J = 7.6, 6.6 Hz, 1H), 7.68 (br, 1H), 7.75-7.80 (m, 2H), 7.84-7.86 (m, 2H), 7.93 (dd, J = 7.2, 7.2 Hz, 1H), 8.00-8.02 (m, 2H), 9.14 (s, 1H), 9.71 (s, 1H);
¹⁹F NMR (acetone-d₆) δ -116.7 (1F), -119.8 (1F);
HRMS (ESI⁺) m/z 494.2248 (calcd. for C₂₈H₃₀F₂N₃O₃⁺, 494.2250, [M+H]⁺).

### Example 8

### Production of Compound CBT-389

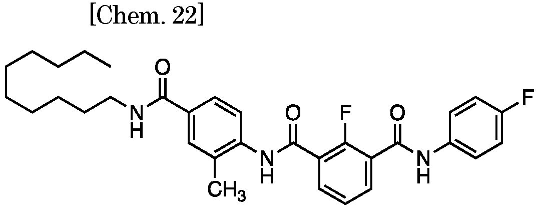

Production was performed according to the above-described general production method using the compound of Formula (S2) (41.1 mg, 0.100 mmol) as the carboxylic acid, 1-aminodecane (39 µL, 0.20 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (11.4 mg, 19.2 mmol, 19.2% yield). Purification was performed by recrystallization (THF/hexane).

¹H NMR (acetone-d₆) δ 0.87 (t, J = 7.2 Hz, 3H), 1.29-1.41 (m, 14H), 1.59-1.63 (m, 2H), 2.41 (s, 3H), 3.39 (dt, J = 7.2, 6.4 Hz, 2H), 7.16 (dd, J = 9.2, 8.4 Hz, 2H), 7.47 (dd, J = 8.0, 7.6 Hz, 1H), 7.64 (br, 1H), 7.76-7.80 (m, 2H), 7.84-7.87 (m, 2H), 7.93 (dd, J = 6.8, 6.0 Hz, 1H), 8.01-8.03 (m, 2H), 9.13 (d, J = 6.8 Hz, 1H), 9.70 (s, 1H);
¹⁹F NMR (acetone-d₆) δ -116.7 (1F), -119.8 (1F);
HRMS (ESI⁺) m/z 550.2876 (calcd. for C₃₂H₃₈F₂N₃O₃⁺, 550.2876, [M+H]⁺).

### Example 9

### Production of Compound CBT-390

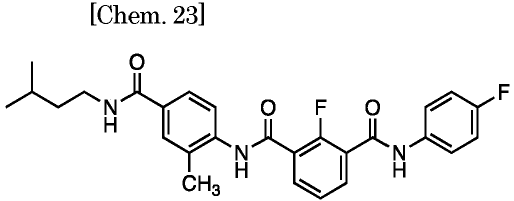

Production was performed according to the above-described general production method using the compound of Formula (S2) (41.1 mg, 0.100 mmol) as the carboxylic acid, 3-methyl-1-butanamine (23 µL, 0.20 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (9.2 mg, 18 µmol, 18% yield). Purification was performed by recrystallization (THF/hexane).

¹H NMR (acetone-d₆) δ 0.93 (s, 3H), 0.95 (s, 3H), 1.52 (dt, J = 6.8, 7.2 Hz, 2H), 1.66-1.71 (m, 1H), 2.41 (s, 3H), 3.42 (dt, J = 6.0, 8.4 Hz, 2H), 7.08 (s, 1H), 7.17 (dd, J = 8.8, 8.8 Hz, 2H), 7.48 (dd, J = 8.0, 8.0 Hz, 1H), 7.64 (br, 1H), 7.76-7.80 (m, 2H), 7.84-7.87 (m, 2H), 7.94 (dd, J = 6.8, 7.6 Hz, 1H), 8.01-8.03 (m, 2H), 9.12 (d, J = 6.0 Hz, 1H), 9.68 (s, 1H);
¹⁹F NMR (acetone-d₆) δ -116.7 (1F), -119.8 (1F);
HRMS (ESI⁺) m/z 480.2093 (calcd. for C₂₇H₂₈F₂N₃O₃⁺, 480.2093, [M+H]⁺).

### Example 10

### Production of Compound CBT-391

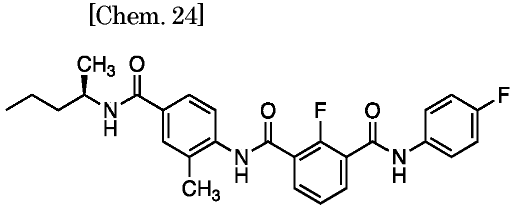

Production was performed according to the above-described general production method using the compound of Formula (S2) (41.1 mg, 0.100 mmol) as the carboxylic acid, (S)-1-methyl-1-butanamine (23 µL, 0.20 mmol, 2.0 equivalent ) as the amine compound, and N,N-diisopropylethylamine (35 µL, 0.20 mmol, 2.0 equivalents) instead of N,N-diisopropylethylamine (1.0 equivalent), and thus, a compound was produced (34.1 mg, 71.1 µmol, 71.1% yield). Purification was performed by recrystallization (THF/hexane).

¹H NMR (acetone-d₆) δ 0.90-0.94 (m, 6H), 1.15-1.22 (m, 1H), 1.46-1.52 (m, 1H), 1.68-1.73 (m, 1H), 2.42 (s, 3H), 3.17-3.24 (m, 1H), 3.31-3.37 (m, 1H), 7.16 (dd, J = 9.2, 8.8 Hz, 2H), 7.47 (dd, J = 7.6, 8.0 Hz, 1H), 7.67 (br, 1H), 7.76-7.81 (m, 2H), 7.84-7.87 (m, 2H), 7.93 (dd, J = 6.8, 6.4 Hz, 1H), 8.01-8.03 (m, 2H), 9.14 (d, J = 6.4 Hz, 1H), 9.70 (s, 1H);
¹⁹F NMR (acetone-d₆) δ -116.7 (1F), -119.8 (1F);
HRMS (ESI⁺) m/z 480.2093 (calcd. for C₂₇H₂₈F₂N₃O₃⁺, 480.2093, [M+H]⁺).

### Example 11

### Production of Compound CBT-400

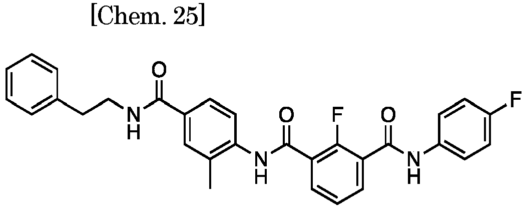

Production was performed according to the above-described general production method using the compound of Formula (S2) (41.1 mg, 0.100 mmol) as the carboxylic acid, 2-phenethylamine (25 µL, 0.20 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (45.1 mg, 87.8 µmol, 87.8% yield). Purification was performed by silica gel column chromatography (hexane/ethyl acetate = 7/3 to 3/7).

¹H NMR (acetone-d₆) δ 2.41 (s, 3H), 2.93 (t, J = 6.4 Hz, 2H), 3.63 (dt, J = 6.4, 6.8 Hz, 2H), 7.15-7.22 (m, 3H), 7.28-7.32 (m, 4H), 7.48 (dd, J = 5.6, 7.6 Hz, 1H), 7.74-7.79 (m, 3H), 7.84-7.87 (m, 2H), 7.94 (dd, J = 6.8, 7.2 Hz, 1H), 8.03-8.04 (m, 2H), 9.13 (s, 1H), 9.70 (s, 1H);
¹⁹F NMR (acetone-d₆) δ -116.8 (1F), -119.7 (1F);
HRMS (ESI⁺) m/z 514.1942 (calcd. for C₃₀H₂₆F₂N₃O₃⁺, 514.1937, [M+H]⁺).

### Example 12

### Production of Compound CBT-401

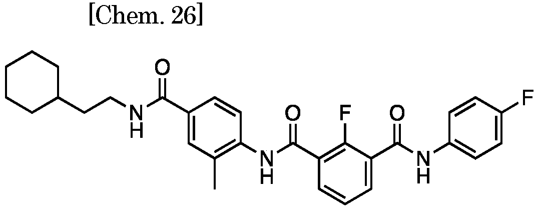

Production was performed according to the above-described general production method using the compound of Formula (S2) (41.1 mg, 0.100 mmol) as the carboxylic acid, 2-cyclohexylethan-1-amine (29 µL, 0.20 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (21.2 mg, 40.8 µmol, 40.8% yield). Purification was performed by recrystallization (chloroform/ethyl acetate).

¹H NMR (DMSO-d₆) δ 0.90 (tt, J = 10.4, 11.6 Hz, 2H), 1.08-1.30 (m, 4H), 1.40-1.43 (m, 2H), 1.60-1.74 (m, 5H), 2.33 (s, 3H), 3.27-3.28 (m, 2H), 7.22 (dd, J = 8.8, 8.8 Hz, 2H), 7.45 (dd, J = 7.6, 7.6 Hz, 1H), 7.61-7.87 (m, 7H), 10.08 (s, 1H), 10.64 (s, 1H);
¹⁹F NMR (DMSO-d₆) δ -116.4 (1F), -118.3 (1F);
HRMS (ESI⁺) m/z 520.2407 (calcd. for C₃₀H₃₂F₂N₃O₃⁺, 520.2406, [M+H]⁺).

### Example 13

### Production of Compound CBT-402

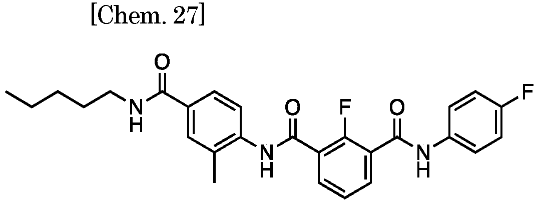

Production was performed according to the above-described general production method using the compound of Formula (S2) (41.1 mg, 0.100 mmol) as the carboxylic acid, n-pentylamine (23 µL, 0.20 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (35.5 mg, 74.0 mmol, 74.0% yield). Purification was performed by recrystallization (ethyl acetate).

¹H NMR (DMSO-d₆) δ 0.88 (t, J = 6.8 Hz, 3H), 1.15-1.19 (m, 1H), 1.30-1.31 (m, 3H), 1.50-1.54 (m, 1H), 2.32 (s, 3H), 3.24 (dt, J = 6.8, 6.4 Hz, 2H), 7.22 (dd, J = 9.2, 8.8 Hz, 2H), 7.45 (dd, J = 7.6, 7.6 Hz, 1H), 7.62 (d, J = 8.0 Hz, 1H), 7.70 (d, J = 8.8 Hz, 1H), 7.74-7.77 (m, 3H), 7.80-7.89 (m, 2H), 8.39 (t, J = 6.0 Hz, 1H), 10.05 (s, 1H), 10.60 (s, 1H);
¹⁹F NMR (DMSO-d₆) δ -116.6 (1F), -118.3 (1F);
HRMS (ESI⁺) m/z 486.2093 (calcd. for C₂₇H₂₈F₂N₃O₃⁺, 486.2093, [M+H]⁺).

### Example 14

### Production of Compound CBT-403

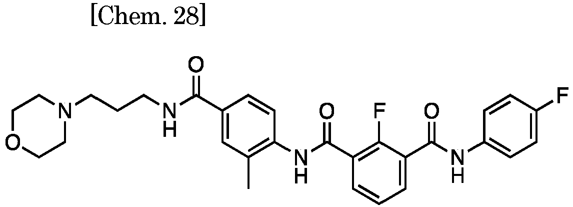

Production was performed according to the above-described general production method using the compound of Formula (S2) (41.1 mg, 0.100 mmol) as the carboxylic acid, 3-(morpholino)propanamine (29 µL, 0.20 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (25.4 mg, 47.3 µmol, 47.3% yield). Purification was performed by recrystallization (ethyl acetate).

¹H NMR (CDCl₃) δ 1.77 (m, 2H), 2.25 (s, 3H), 2.61 (m, 6H), 3.35 (m, 2H), 3.76 (m, 4H),7.03 (dd, J = 7.6, 6.8 Hz, 2H), 7.33 (dd, J = 7.6, 5.2 Hz, 1H), 7.62-7.68 (m, 4H), 7.95-8.20 (m, 5H), 8.96 (s, 1H);
¹⁹F NMR (CDCl₃) δ -116.0 (1F), -116.9 (1F);
HRMS (ESI⁺) m/z 537.2309 (calcd. for C₂₉H₃₁F₂N₃O₃⁺, 537.2308, [M+H]⁺).

### Example 15

### Production of Compound CBT-404

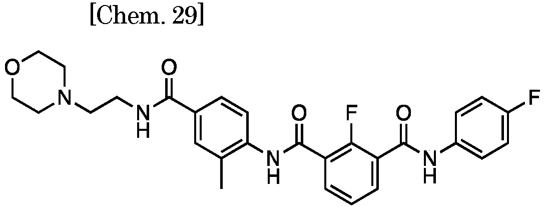

Production was performed according to the above-described general production method using the compound of Formula (S2) (41.1 mg, 0.100 mmol) as the carboxylic acid, 2-(morpholino)ethylamine (26.8 mg, 0.206 mmol, 2.0 equivalents) as the amine compound, and N,N-diisopropylethylamine (35 µL, 0.20 mmol, 2.0 equivalents) instead of N,N-diisopropylethylamine (1.0 equivalent), and thus, a compound was produced (49.2 mg, 94.2 µmol, 94.2% yield). Purification was performed by recrystallization (chloroform/hexane).

¹H NMR (CDCl₃) δ 2.26 (s, 3H), 2.50-2.55 (m, 6H), 3.40-3.41 (m, 2H), 3.71 (m, 4H), 6.86 (br, 1H), 7.04 (dd, J = 8.8, 8.8 Hz, 2H), 7.32 (dd, J = 7.6, 7.6 Hz, 1H), 7.50-7.55 (m, 2H), 7.96 (m, 1H), 8.10-8.21 (m, 3H), 8.96 (d, J = 5.6 Hz, 1H);
¹⁹F NMR (CDCl₃) δ -116.2 (1F), -116.8 (1F);
HRMS (ESI⁺) m/z 523.2151 (calcd. for C₂₈H₂₉F₂N₃O₃⁺, 523.2155, [M+H]⁺).

### Experiment for Measuring Anti-HBV Activity Using Hepatitis B Virus-Producing Cultured Cells

Hep2.2.15.7 cells were spread in a 96-well plate (manufactured by TPP) at a density of 1 × 10⁵ cells/well and cultured at 37°C and 5% CO₂ using a culture medium containing DMEM/F12-GlutaMAX (manufactured by Thermo Fisher Scientific Inc.), 10% fetal bovine serum, 100 U/ml penicillin, 100 µg/ml streptomycin (manufactured by Thermo Fisher Scientific Inc.), and 5 µg/ml insulin (manufactured by Wako Pure Chemical). A test compound was added to the culture, and cells in three wells per condition were cultured. After 3 days, replacement with a medium containing the test compound was performed. After 6 days, cell viability was determined by quantitatively measuring intracellular mitochondrial activity. This was performed by measuring absorbance at 490 nm using Cell Proliferation Kit II (XTT) (manufactured by Roche) and a plate reader, EnSight (manufactured by PerkinElmer). At the same time, HBV-derived DNA was extracted and purified from the cell culture medium using Agencourt Genfind v2 (manufactured by Beckman-Coulter). The DNA solution (2 µL), Probe qPCR Mix (manufactured by TAKARA) (5 µL), and a 100 µM primer (0.02 µL) (HBV DNA Fr: ACTCACCAACCTCCTGTCCT (SEQ ID NO: 1), HBV DNA Rv: GACAAACGGGCAACATACCT (SEQ ID NO: 2), a 100 µM probe (0.01 µL), and HBV DNA: [FAM]TATCGCTGGATGTGTCTGCGGCGT[TAM] (SEQ ID NO: 3)) were mixed, qPCR reaction was performed at 95°C for 1 minute, followed by 50 cycles, each consisting of "95°C for 10 seconds and 60°C for the subsequent 30 seconds", and the HBV DNA level (HBV gene copy number) was quantitatively determined by detecting signals using CFX96 Touch Real-Time PCR Detection System (manufactured by Bio-rad).

### Experiment for Measuring Anti-HBV Activity Using Hepatitis B Virus-Infected Primary Cultured Human Hepatocytes

Fresh human hepatocytes derived from PXB mice (PXB cells; manufactured by PhoenixBio Co., Ltd.) were spread in a collagen-coated 96-well plate (manufactured by FALCON) at a density of 0.7 × 10⁵ cells/well and cultured for 7 days at 37°C and 5% CO₂ using a dHCGM medium (manufactured in house). HBV genotype C (manufactured by PhoenixBio Co., Ltd.) or HBV genotype D (derived from Hep2.2.15.7 cells; manufactured in house) was added to the culture medium together with 4% PEG 8000 at a final density of 3 × 10⁵ copies/well or 3 × 10⁷ copies/well, respectively. The medium was replaced after 1 day, and every third or fourth day thereafter. A test compound was added after 28 days, and cells in three wells per condition were cultured. Then, replacement with a medium containing the test compound was also performed after 3 or 4 days, and after 7 days, total RNA was extracted and purified from the cells using Agencourt RNAdvance (manufactured by Beckman-Coulter), and reverse transcribed using PrimeScript RT reagent Kit (Perfect Real Time). The cDNA solution (2 µL), TB Green Premix Ex Taq II (TIi RNaseH Plus) (manufactured by TAKARA) (5 µL), and a 100 µM primer (0.02 µL) (pgRNAFr: CTCAATCTCGGGAATCTCAATGT (SEQ ID NO: 4) and pgRNARv: GGATAGAACCTAGCAGGCATAAT (SEQ ID NO: 5)) were mixed, qPCR reaction was performed at 95°C for 1 minute, followed by 50 cycles, each consisting of "95°C for 10 seconds and 60°C for the subsequent 30 seconds", and the pgRNAlevel (pgRNA copy number) was quantitatively determined by detecting signals using CFX96 Touch Real-Time PCR Detection System (manufactured by Bio-rad).

The results of measuring the anti-HBV activity of compounds using PXB cells or Hep2.2.15.7 cells are summarized in Table 3 below. The anti-HBV activity was measured as follows: the HBV gene copy number in the cells or in the culture medium was calculated, with the copy number in a DMSO-treated sample taken as 0% inhibition and 0 copy number as 100% inhibition, and the IC₅₀ was obtained.

**[Table 3]**

| 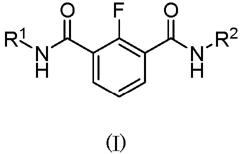 | | | |
|---|---|---|---|
| CBT Number | Structure of R1 | IC50 (HBV DNA from Hep2.2.15.7) µM | IC50 (pgRNA in PXB cells) µM |
| CBT-209 | 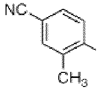 | 0.039 | - |
| CBT-289 | 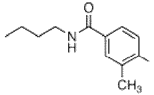 | 0.074 | 2.48 |
| CBT-383 | 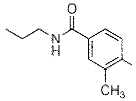 | 0.91 | 2.33 |
| CBT-384 | 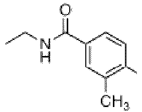 | 0.075 | 16.5 |
| CBT-385 | 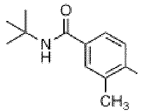 | 0.18 | > 100 |
| CBT-387 | 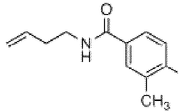 | 0.041 | 3.41 |
| CBT-388 | 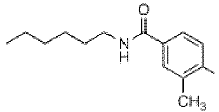 | 0.18 | 4.59 |
| CBT-389 | 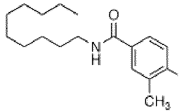 | 0.47 | 100 |
| CBT-390 | 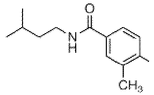 | 0.061 | 0.73 |
| CBT-391 | 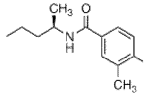 | 0.095 | 2.48 |
| CBT-078 (Comparative) | 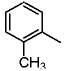 | 0.27 | - |

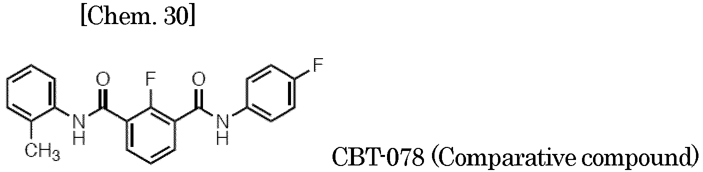

As shown in the table above, the compounds of Formula (I) exhibited HBV inhibitory activity. More specifically, the conventional capsid allosteric modulator CBT-078 did not have the effect of suppressing pgRNA or cccDNAin the hepatitis B virus-infected primary cultured human hepatocytes, whereas the compounds of Formula (I) were confirmed to have the effect of suppressing pgRNA and cccDNA. Although conventional nucleotide/nucleoside analog preparations cannot eliminate cccDNA, and it is difficult to completely cure hepatitis B, the compounds of Formula (I) have the effect of suppressing cccDNA and are expected to improve the therapeutic efficacy for hepatitis B.

### Experiment for Measuring Mouse Blood Drug Concentrations

A compound was dissolved in a mixture of ethanol and polysorbate 80 (1:1) to make 10 mg/mL, and the resulting mixture was diluted with saline to 1 mg/mL and orally administered to mice (ICR strain, males, 6-10 weeks old) using a sonde. Blood was collected from the tail vein at 0.25, 0.5, 1, 2, 4, and 7 hours after administration, and plasma was obtained. An internal standard (ritonavir) was added to 10 µL of plasma, and a deproteinized sample was used for measurement. The measurement was performed according to a liquid chromatography-tandem mass spectrometry method using a Shimadzu LCMS-8050 system. The analytical conditions were as follows. A COSMOSIL 3C18-MS-II (2.0 ID × 50 mm; manufactured by Nacalai Tesque, Inc.) column was used. The column temperature was 40°C, and the autosampler temperature was 4°C. The mobile phase was a combination of Phase A: water/acetonitrile = 9/1 (v/v) and Phase B: acetonitrile, and the flow rate was 0.6 mL/min. 2 µL of the sample was injected. The gradient elution conditions were 0 to 0.9 min, 5% B; 0.9 to 2.1 min, 5 to 95% B; 2.1 to 5.5 min, 95% B; 5.5 to 5.51 min, 95 to 5% B; and 5.51 to 6.0 min, 5% B. The cleaning solution used was an isopropanol/methanol/acetonitrile/water = 1/1/1/1 (v/v/v/v, %) solution containing 0.1% formic acid. The ionization method was ESI, and the collected data was processed using LabSolutions software (Ver. 5.97 SP1, Shimadzu Corporation).

The mouse blood drug concentrations of the compounds were summarized in Table 4.

**[Table 4]**

| CBT Compound | Maximum blood concentration (ng/ml) | Time to reach maximum blood concentration (min) | Elimination half-life (min) |
|---|---|---|---|
| CBT-193 | 4.7 | 30 | ND |
| CBT-213 | 18 | 60 | 118 |
| CBT-215 | 190 | 60 | 105 |
| CBT-209 | 250 | 120 | 825 |
| CBT-300 | 5700 | 30 | 126 |
| CBT-301 | 33 | 240 | ND |
| CBT-078 (Comparative) | 710 | 30 | 32 |

As shown in the table above, it was confirmed that the compounds of Formula (I) have a slow absorption rate and a slow elimination rate.

### Example 16

### Production of Compound CBT-209 Labeled with ¹⁸F (Compound [¹⁸F]CBT-209)

A compound CBT-398 was first synthesized according to the following scheme. The reagents used were purchased and used as is. The method for synthesizing monomethyl 2-fluoroisophthalic acid (S3) was as described in the paper (J. Med. Chem. 2009, 52, 1518-1521).

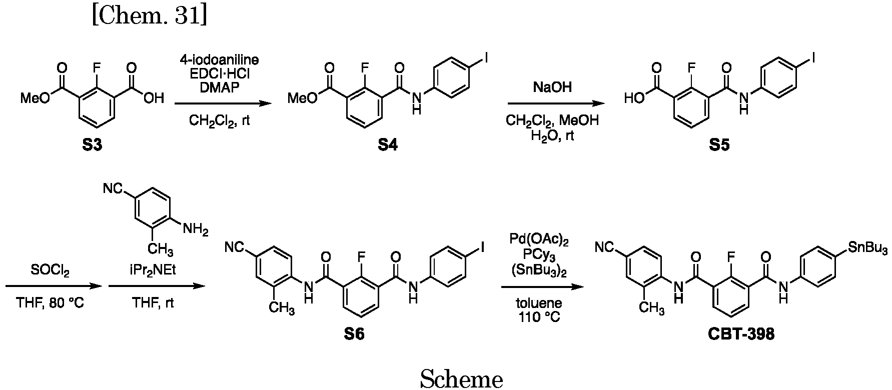

### (1) Production of Compound of Formula (S4)

4-Iodoaniline (1.00 g, 4.57 mmol, 1.5 equivalents) was added to a dichloromethane solution (30 mL) containing the compound of Formula (S3) (594 mg, 3.00 mmol, 1 equivalent), 1-(3-dimethylaminopropyl)-3-ethylcarbodiimide hydrochloride (EDCI-HCl, 867 mg, 4.52 mmol, 1.5 equivalents), and 4-dimethylaminopyridine (DMAP, 370 mg, 3.03 mmol, 1 equivalent), and the mixture was stirred at room temperature for 20 hours. A hydrochloric acid solution (1 mol/L, 20 mL) was added to the mixture, followed by stirring, and the resulting mixture was then extracted twice with chloroform. Combined organic phases were washed with a hydrochloric acid solution and brine, and then dehydrated with anhydrous sodium sulfate. After the solvent was distilled off from the organic phases under reduced pressure, acetone was added to the solid residue, which was then heated to 70°C and dissolved in acetone. After cooling this acetone solution, purification was performed by silica gel column chromatography (n-hexane/EtOAc = 9/1 to 6/4). Thus, a compound of Formula (S4) was obtained (1.09 g, 2.74 mmol, 91.2% yield).

¹H NMR (CDCl₃) δ 3.98 (s, 3H), 7.40 (dd, J_{H-H}= 8.0, 8.0 Hz, 1H), 7.44-7.47 (AA'BB', 2H), 7.68-7.70 (AA'BB', 2H), 8.12 (ddd, J_{H-H} = 8.0, 2.0 Hz, J_{H-F} = 8.0 Hz 1H), 8.34 (ddd, J_{H-H}= 8.0, 2.0 Hz, J_{H-F} = 8.0 Hz 1H), 8.41 (d, J_{H-F} = 15.0 Hz, 1H);
¹⁹F NMR (CDCl₃) δ -112.7 (d, JF-H = 15.0 Hz, 1F);
HRMS (ESI⁺) m/z 399.9839 (calcd. for C₁₀H₁₀FO₄⁺, 399.9840, [M+H]⁺).

### (2) Production of Compound of Formula (S5)

The compound of Formula (S4) (1.09 g, 2.73 mmol, 1 equivalent) was dissolved in a solvent mixture of dichloromethane (20 mL), methanol (5 mL), and water (5 mL). To this solution was added sodium hydroxide (3.28 g, 8.21 mmol, 30 equivalents), and the obtained mixture was stirred at room temperature for 23 hours. A hydrochloric acid solution (1 mol/L, 100 mL) was added to the mixture, and the resulting white solid was collected by filtration under reduced pressure. Thus, a compound of Formula (S5) was obtained (980 mg, 2.54 mmol, 93.2% yield).

¹H NMR (acetone-d₆) δ 7.44 (dd, J_{H-H} = 8.0, 8.0 Hz, 1H), 7.66-7.74 (m, 4H), 7.96-8.00 (m, 1H), 8.07-8.11 (m, 1H), 9.70 (s, 1H);
¹⁹F NMR (acetone-d₆) δ -113.2 (1F);
HRMS (ESI⁻) m/z 383.9540 (calcd. for C₁₄H₈FINO₃⁻, 383.9538, [M-H]⁻)

### (3) Production of Compound of Formula (S6)

Thionyl chloride (1.45 mL, 20.0 mmol, 20 equivalents) was added to a THF solution (10 mL) containing the compound of Formula (S5) (790 mg, 2.05 mmol, 1 equivalent), and the obtained mixture was heated to 80°C and stirred for 2 hours. The mixture was then cooled to room temperature and dried under reduced pressure. To the resulting residue were added THF (10 mL), 3-methyl-4-aminobenzonitrile (397 mg, 3.00 mmol, 1.5 equivalents), and N,N-diisopropylethylamine (0.696 mL, 4.00 mmol, 2 equivalents), and the obtained mixture was stirred at room temperature for 20 hours. A hydrochloric acid solution (1 mol/L, 10 mL) was added to the mixture, followed by stirring, and the resulting mixture was extracted three times with ethyl acetate. Combined organic phases were washed sequentially with a hydrochloric acid solution, an aqueous solution of sodium hydrogen carbonate, and brine, and then dehydrated with anhydrous sodium sulfate. After the solvent was distilled off from the organic phases under reduced pressure, acetone was added to the solid residue, which was then heated to 70°C and dissolved in acetone. After cooling the acetone solution, a small amount of hexane was added to precipitate the solid. The solid was collected by filtration. Thus, a compound of Formula (S6) was obtained (647 mg, 1.30 mmol, 64.8% yield).

¹H NMR (acetone-d₆) δ 2.45 (s, 3H), 7.49 (dd, J_{H-H} = 8.0, 8.0 Hz, 2H), 7.66-7.70 (m, 4H), 7.72-7.75 (m, 2H), 7.92-7.96 (m, 1H), 8.01-8.06 (m, 1H), 8.24 (d, J_{H-H}= 8.8 Hz, 1H), 9.33 (s, 1H), 9.86 (s, 1H);
¹⁹F NMR (acetone-d₆) δ -116.5 (1F);
HRMS (ESI⁻) m/z 498.0122 (calcd. for C₂₂H₁₁FIN₃O₂⁻, 498.0120, [M-H]⁻)

### (4) Production of Compound CBT-398

Hexabutylditin (1.14 mL, 2.24 mmol, 2.5 equivalents) was added to a toluene solution (9 mL) containing the compound of Formula (S6) (449 mg, 0.90 mmol, 1 equivalent), palladium(II) acetate (12.5 mg, 55.7 µmol, 0.05 equivalents), and tricyclohexylphosphine (25.3 mg, 90.2 µmol, 0.1 equivalents), and the resulting mixture was heated to 110°C and stirred for 24 hours. The mixture was cooled to room temperature, and water (5 mL) was added, followed by stirring. Then, the mixture was extracted three times with ethyl acetate. Combined organic phases were washed with brine and then dehydrated with anhydrous sodium sulfate. After the solvent was distilled off from the organic phases under reduced pressure, the residue was purified by silica gel column chromatography (n-hexane/EtOAc = 9/1 to 7/3). Thus, a compound CBT-398 was obtained (442 mg, 0.667 mmol, 74.1% yield).

¹H NMR (acetone-d₆) δ 0.89 (t, J_{H-H} = 7.6 Hz, 9H), 1.03-1.20 (m, 6H), 1.31-1.40 (m, 6H), 1.59-1.68 (m, 6H), 2.46 (s, 3H), 7.47-7.52 (m, 3H), 7.67-7.70 (m, 2H), 7.78-7.80 (AA'BB', 2H), 7.93-7.97 (m, 1H), 8.02-8.28 (m, 1H), 8.29 (d, J_{H-H}= 8.4 Hz, 1H), 9.25 (d, J_{H-F} = 4.8 Hz, 1H), 9.57 (s, 1H);
¹⁹F NMR (acetone-d₆) δ -116.6 (1F);
HRMS (ESI⁻) m/z 662.2208 (calcd. for C₃₄H₄₁FIN₃O₂Sn⁻, 662.2210, [M-H]⁻)

### (5) Production of Compound [¹⁸F]CBT-209

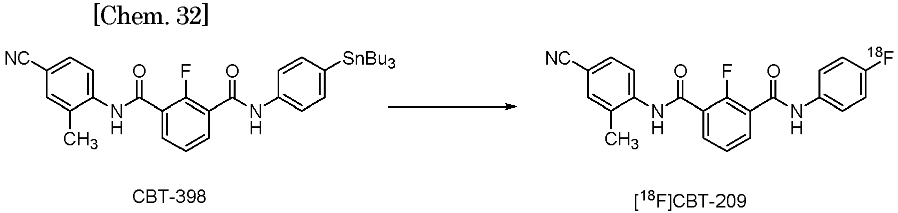

A sealed vial and a reaction V-vial were provided. The reaction was carried out using oven-dried devices, and before the start of the reaction, a flow path was dried by repeating decompression and pressurization with helium gas while heating a reaction vessel at 150°C.

A N,N-dimethylacetamide solution (300 µL) containing the compound CBT-398 (5.3 mg, 8.0 µmol) and a N,N-dimethylacetamide solution (300 µL) containing copper(II) trifluoromethanesulfonate (8.7 mg, 24 µmol) and pyridine (9.7 µL, 120 µmol) were prepared 10 minutes before the completion of the production of [¹⁸F] fluoride ions.

[¹⁸O]H₂O (2 mL, ¹⁸O: > 98 atom%, Taiyo Nippon Sanso Corp., F03-0027) was irradiated with a proton laser (35 µA) for 40 minutes using a cyclotron (CYPRIS HN-12S) manufactured by Sumitomo Heavy Industries, Ltd., to thereby synthesize [¹⁸F] fluoride ions (approximately 40 GBq). The synthesized [¹⁸F] fluoride ions were transferred into the sealed vial placed inside a synthesizer for labeling. After confirming that an RI detector that was located adjacent to the sealed vial showed the maximum value, the sealed vial was pressurized with nitrogen gas at a flow rate of 500 mL/min to transfer [¹⁸O]H₂O containing [¹⁸F] fluoride ions and allow [¹⁸O]H₂O to pass through an ion exchange resin (Waters, 186004540), and [¹⁸F] fluoride ions were captured. After confirming that an RI detector that was located adjacent to the ion exchange resin showed the maximum value, pressurization with nitrogen gas was stopped, and the [¹⁸F] fluoride ions adsorbed on the ion exchange resin were eluted with a solvent mixture of acetonitrile (1 mL) and an aqueous solution (500 µL) containing potassium trifluoromethanesulfonate (5 mg) and potassium carbonate (50 µg) and transferred into the reaction Vvial. The eluted [¹⁸F] potassium fluoride solution was decompressed at 130°C for 5 minutes to distill off water. To the small amount of remaining solution was added acetonitrile (600 µL), and decompression was performed at 150°C for 5 minutes to azeotrope the water content. This was repeated once more to prepare anhydrous [¹⁸F] potassium fluoride in the reaction V-vial.

AN,N-dimethylacetamide solution (300 µL) containing the compound CBT-398 (5.3 mg, 8.0 µmol) was introduced into the reaction Vvial. Subsequently, a N,N-dimethylacetamide solution (300 µL) containig copper(II) trifluoromethanesulfonate (8.7 mg, 24 µmol) and pyridine (9.7 µL, 120 µmol) was also introduced into the reaction V-vial, and the reaction V-vial was heated at 140°C for 30 minutes. After cooling the V-vial to room temperature, a solvent mixture of acetonitrile (500 µL) and water (500 µL) was introduced into the V-vial, and the resulting mixture was filtered through a membrane filter (made of PVDF, 0.2 µm, diameter 13 mm, GE Healthcare Life Science, 6779-1302) and then introduced into a semi-preparative HPLC. A fraction containing the compound [¹⁸F]CBT-209 was separated by referring to a UV sensor and an RI detector attached to the HPLC. This fraction was transferred into a flask attached to an evaporator installed in a hot cell, heated at reduced pressure, and distilled under reduced pressure for 3 minutes. After the interior of the flask was brought to normal temperature and pressure, a dilute solution for administration (saline:propylene glycol:Tween 80 = 100:10:1, 0.7 mL) was introduced into the flask and stirred by rotation for 40 seconds. The resulting solution was transferred into an administration vial through a membrane filter (made of PVDF, 0.45 µm, diameter 33 mm, Merck, SLHV033NB). Thus, a solution of the compound [¹⁸F]CBT-209 was obtained.

The total radioactivity of the obtained solution of the compound [¹⁸F]CBT-209 was measured using a dose calibrator (Capintec, Inc., 5130-3235). The purity test was performed using an HPLC equipped with a UV detector and an RI detector. Furthermore, the non-radioactivity was determined by calculating, with the density of the obtained solution taken as 1 g/mL, the concentration of the compound [¹⁸F]CBT-209 based on its UV absorption in the HPLC, and then calculating its ratio to the total radioactivity. Detailed analytical conditions of the HPLC in the semi-preparative and purity tests, along with examples of the results, are shown below.
Conditions for Semi-Preparative HPLC
   Column: COSMOSIL 5C18-MS-II (Nacalai Tesque, Inc.) 10 × 20 mm and 10 × 250 mm columns were connected and used;
   Flow rate: 5 mL/min; Mobile phase: acetonitrile/H₂O = 48:52;
   UV detection wavelength: 254 nm; Retention time: 24.9 minutes
Conditions for Analytical HPLC
   Column: COSMOSIL 5C18-MS-II (Nacalai Tesque, Inc.) 4.6 × 150 mm;
   Flow rate: 1 mL/min; Mobile phase: acetonitrile/H₂O = 50:50;
   UV detection wavelength: 254 nm; Retention time: 7.3 minutes
Analysis Results of [¹⁸F]CBT-209 Solution (Data at 89 Minutes after [¹⁸F] Fluoride Ion Synthesis)
   Total radioactivity: 2190 MB1; Specific radioactivity: 50.0 GBq/µmol; Volume of solution: 3.8 mL;
   Weight concentration of [¹⁸F]CBT-209: 4.50 µg/mL; Chemical purity: > 99.9%; Radiochemical purity: > 99.9%

### Mouse PET Imaging

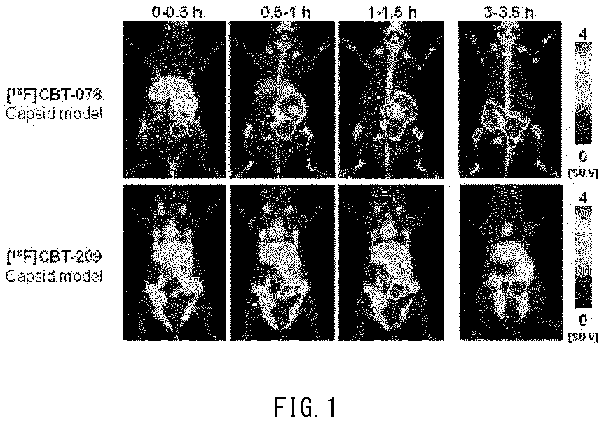

Model mice with forced expression of capsid were prepared as HBV non-infectious model animals and used for the experiment. The model mice with forced expression of capsid were each prepared by dissolving 40 µg of the plasmid pCI-HBc, which encodes only the capsid protein HBcAg, into a PBS solution, the volume of which was 1/10 of the body weight per mouse plus 100 µL (for example, 2.1 mL in total for a mouse weighing 20 g), and administering the resulting solution to a 6-week-old male BALB/c mouse through hydrodynamic injection. Approximately 24 hours after administration of the PBS solution, the mouse was retained on an imaging bed of a small-animal PET system (Siemens, microPET Focus 220) under 1.5% isoflurane inhalation anesthesia, and the compound [¹⁸F]CBT-078, as 50 MBq / 0.03 nmol / 100 µL saline (containing 1% Tween 80), or the compound [¹⁸F]CBT-209, as 20 MBq / 0.01 nmol / PBS 100 µL saline (containing 1% Tween 80), was administered into the tail vein. Imaging was started at the same time as administration, and after 90 minutes of data collection, the mouse was temporarily awakened, and then, PET imaging was performed again under 1.5% isoflurane inhalation anesthesia for 30 minutes, starting from 3 hours after administration. The collected data were converted to dynamic sinograms and then reconstructed into three-dimensional dynamic images using filtered back projection with a Ramp filter cutoff at the Nyquist frequency. The data collected over individual 30-minute periods were integrated and presented in the results as maximum density projection images (MIP images). FIG. 1 shows the obtained results.

It was confirmed from FIG. 1 that accumulation of F⁻ in bone observed with respect to the compound [¹⁸F]CBT-078 was significantly reduced in the case of the compound [¹⁸F]CBT-209 due to improved *in-vivo* stability, and the retention of hepatic accumulation was improved. On the other hand, in the case of the compound [¹⁸F]CBT-209, changes in systemic distribution, such as in adipose tissue, were also observed compared with the compound [¹⁸F]CBT-078. This suggests accumulation in fat and an improvement in pharmacokinetics due to a controlled-release effect.

Metabolic stability tests for the compound CBT-078 and the compound CBT 209, respectively, were conducted using liver microsomes. The operation was as follows.

10 µL of 50 µM CBT-078 or CBT-209 was added to a microsome reaction solution constituted by 422.5 µL of a 100 mM phosphate buffer (pH 7.4), 5 µL of a 300 mM magnesium chloride solution, and 12.5 µL of 20 mg/mL liver microsomes (human, mouse, and rat, all manufactured by Zeno Tech) and pre-incubated for 15 minutes. Then, 50 µL of a NADPH regeneration system (85 mM D-glucose-6-phosphate, 10 mM β-nicotinamide adenine dinucleotide phosphate, oxidized form, and 10 U/mL glucose-6-phosphate dehydrogenase, all manufactured by Oriental Yeast Co., Ltd.) was added and mixed, and incubation was started. The incubation starting point was set to 0 minutes of reaction. At 0, 30, and 60 minutes of reaction, a 50-µL fraction of the reaction solution was collected into 200 µL of an ice-cooled 0.5 µM propranolol/acetonitrile solution, and the reaction was stopped. The obtained preparative solution was transferred to a protein removal plate (manufactured by Phenomenex), and after suction filtration through a manifold for 96-well plates, the filtrate was collected into a 96-well plate for collection. 100 µL of the collected filtrate was transferred to a 96-well plate and dried in a nitrogen-blowing high-speed parallel concentrator (Turbo Vap 96, manufactured by Biotage). After dissolving the dried sample with the initial mobile phase, the target compound was quantified by liquid chromatography-tandem mass spectrometry (LC-MS/MS). The peak area value ratio of CBT-078 or CBT-209 in the sample to propranolol (internal standard) was calculated, and the metabolic stability was evaluated based on the "residual rate (%) = (peak area value ratio at 30 or 60 minutes of incubation / peak area value ratio at 0 minutes of incubation) × 100". As technical replicates, the residual rate was calculated in two independent series for each compound to be evaluated for each animal species.

The configuration of LC-MS/MS is as follows: high-performance liquid chromatography: Nexera X2 UPLC system (manufactured by Shimadzu), column: ACQUITY UPLC BEH C18 Column, 130A, 1.7 µm, 2.1 ×× 50 mm (manufactured by Waters), guard column: ACQUITY UPLC BEH C18 VanGuard Column, 130A, 1.7 µm, 2.1 ×× 50 mm (manufactured by Waters), mobile phase A: 0.1% aqueous formic acid solution/acetonitrile (9:1, v/v), mobile phase B: 0.1% aqueous formic acid solution/acetonitrile (1:9, v/v), gradient conditions: 0 to 1 min; 30% B, 1 to 1.5 min; 30 to 100% B, 1.5 to 3.5 min; 100% B, and 3.5 to 5 min; 30% B, and mass spectrometer: QTRAP 4500 (manufactured by AB Sciex) were used, a measurement target compound was ionized using an ESI method, and target ions were measured by multiple reaction monitoring. Table 5 shows the obtained results.

**[Table 5]**

| Metabolic stability test for compound CBT-078, conducted using liver microsomes | | | |
|---|---|---|---|
| | 0 min | 30 min | 60 min |
| Human | 100% | 77% | 60% |
| | 100% | 77% | 58% |
| Rat | 100% | 53% | 35% |
| | 100% | 52% | 34% |
| Mouse | 100% | 6% | 0% |
| | 100% | 6% | 0% |

| Metabolic stability test for compound CBT-209, conducted using liver microsomes | | | |
|---|---|---|---|
| | 0 min | 30 min | 60 min |
| Human | 100% | 91% | 84% |
| | 100% | 97% | 86% |
| Rat | 100% | 87% | 73% |
| | 100% | 77% | 63% |
| Mouse | 100% | 88% | 70% |
| | 100% | 81% | 72% |

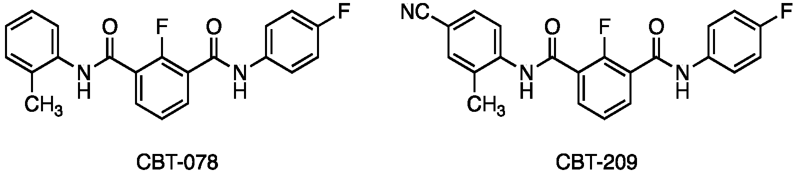

It was confirmed from Table 5 that, in the case of the compound CBT-209, the metabolic stability after hepatic accumulation was improved compared with the compound CBT-078.

### Effect of Suppressing HBsAg, HBeAg, and pgRNA

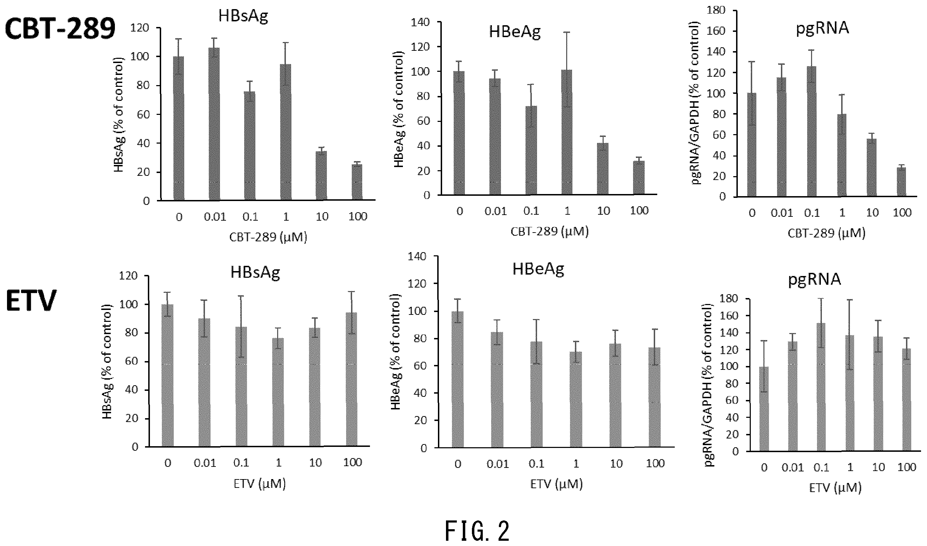

Primary cultured human hepatocytes infected with HBV were treated with the compound CBT-289 or Entecavir (ETV) at concentrations of 0.01 to 100 mM, and the HBsAg and HBeAg levels in the medium and the pgRNAlevel in the cells were measured. FIG. 2 shows the results.

As shown in FIG. 2, the compound CBT-289 suppressed HBsAg, HBeAg, and pgRNAin a concentration-dependent manner. On the other hand, ETV did not suppress them.

### Effect of Suppressing cccDNA and pgRNA

### Method for Measuring the Effect of Suppressing cccDNA

Fresh human hepatocytes derived from PXB mice (PXB cells; manufactured by PhoenixBio Co., Ltd.) were spread in a collagen-coated 96-well plate (manufactured by FALCON) at a density of 0.7 × 10⁵ cells/well, and cultured for 7 days using a dHCGM medium (manufactured in house) at 37°C and 5% CO₂. HBV genotype C (manufactured by PhoenixBio Co., Ltd.) was added to the culture medium together with 4% PEG 8000 at a final density of 3.5 × 10⁵ copies/well, and the medium was replaced after 1 day, and every third or fourth day thereafter. A test compound was added after 28 days, and cells in three wells per condition were cultured. Then, replacement with a medium containing the test compound was also performed after 3 or 4 days, and after 7 days, DNA was extracted and purified from the cells using Agencourt DNAdvance (manufactured by Beckman-Coulter). The DNA solution (2 µL) was mixed with Probe qPCR (manufactured by TAKARA) (5 µL), a 100 µM primer (0.02 µL) (cccDNA Fr: GTGGCTATCCTGCCTTAAT (SEQ ID NO: 9), cccDNA Rv: CAGAGCTGAGGCGGTGTC (SEQ ID NO: 10)), and a 100 µM probe (0.01 µL) (cccDNA: [FAM]AGTTGGCGAGAAAGTGAAAGCCTGC[TAM] (SEQ ID NO: 11)), qPCR reaction was performed at 95°C for 1 minute, followed by 50 cycles, each consisting of "95°C for 10 seconds and 60°C for the subsequent 30 seconds", and the HBV DNA level (HBV gene copy number) was quantitatively determined by detecting signals using CFX96 Touch Real-Time PCR Detection System (manufactured by Bio-rad).

### Method for Measuring the Effect of Suppressing pgRNA

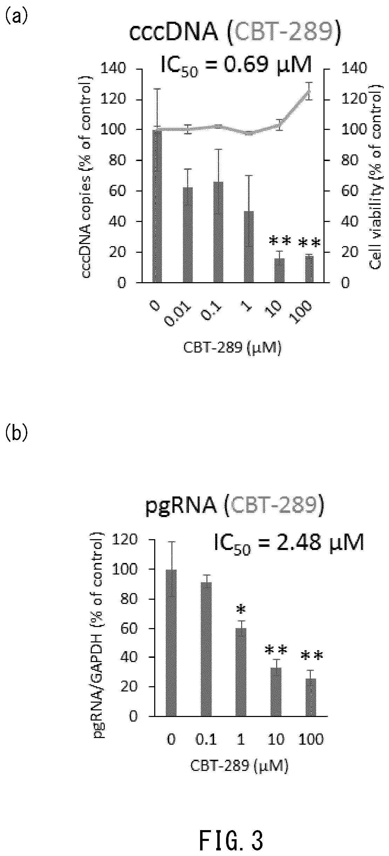

Fresh human hepatocytes derived from PXB mice (PXB cells; manufactured by PhoenixBio Co., Ltd.) were spread in a collagen-coated 96-well plate (manufactured by FALCON) at a density of 0.7 × 10⁵ cells/well, and cultured for 7 days using a dHCGM medium (manufactured in house) at 37°C and 5% CO₂. HBV genotype D (derived from Hep2.2.15.7 cells; manufactured in house) was added to the culture medium together with 4% PEG 8000 at a final density of 3 × 10⁷ copies/well, and the medium was replaced after 1 day, and every third or fourth day thereafter. A test compound was added after 28 days, and cells in three wells per condition were cultured. Then, replacement with a medium containing the test compound was also performed after 3 or 4 days, and after 7 days, total RNA was extracted and purified from the cells using Agencourt RNAdvance (manufactured by Beckman-Coulter), and reverse transcribed using the PrimeScript RT reagent Kit (Perfect Real Time). The cDNA solution (2 µL) was mixed with TB Green Premix Ex Taq II (TIi RNaseH Plus) (manufactured by TAKARA) (5 µL) and a 100 µM primer (0.02 µL) (pgRNA Fr: CTCAATCTCGGGAATCTCAATGT (SEQ ID NO: 4) and pgRNA Rv: GGATAGAACCTAGCAGGCATAAT (SEQ ID NO: 5)), qPCR reaction was performed at 95°C for 1 minute, followed by 50 cycles, each consisting of "95°C for 10 seconds and 60°C for the subsequent 30 seconds", and the pgRNA level (pgRNA copy number) was quantitatively determined by detecting signals using CFX96 Touch Real-Time PCR Detection System (manufactured by Bio-rad). FIG. 3 shows the results.

As shown in FIG. 3, the compound CBT-289 suppressed cccDNA and pgRNA in a concentration-dependent manner.

Experiment for Measuring Anti-HBV Activity of Compound CBT-209 Using Hepatitis B Virus-Infected Human Hepatocytes Chimeric Mice

Human hepatocyte-transplanted chimeric mice (PXB mice <http://phoenixbio.co.jp/pxb/pxb-mouse.html>; manufactured by PhoenixBio Co., Ltd.) were infected with 1 × 10⁷ copies of HBV genotype C (manufactured by PhoenixBio Co., Ltd.) per mouse. After the virus was sufficiently grown to 1 × 10⁸ copies/ml serum, 10 mg/kg of the compound CBT-209 was orally administered twice daily for 21 days (solvent: corn oil) (5 mice per group). Blood was collected from the mice on days 0, 7, 14, and 21 after the start of administration, and the HBV DNA level in the serum was measured.

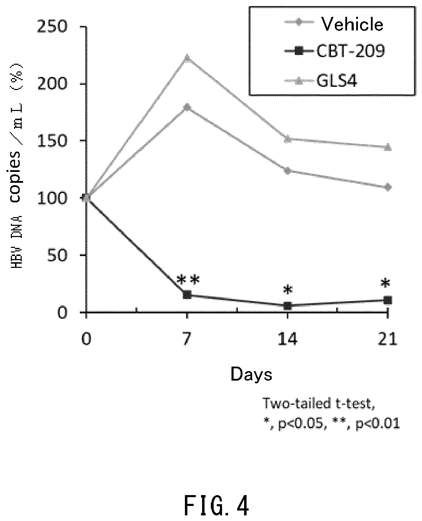

The serum was mixed with HBV Pre-treatment Solution for HBV DNA in Serum and heat-treated at 98°C for 5 minutes to inactivate HBV and expose HBV DNA. PCR reaction and analysis were performed using KUBIX HBV qPCR Kit (KUBIX Inc.) and CFX96 Touch (registered trademark) Real-Time PCR Detection System (Bio-Rad Laboratories, Inc., Hercules, CA, USA). A 10 µL HBV 2 × PCR Solution (Forward primer: CACATCAGGATTCCTAGGACC (SEQ ID NO: 6), Reverse primer: AGGTTGGTGAGTGATTGGAG (SEQ ID NO: 7), Probe: CAGAGTCTAGACTCGTGGTGGACTTC (SEQ ID NO: 8)) was mixed with 10 µL of a heat-treated standard or a sample to be measured. PCR reaction was performed at 95°C for 2 minutes, followed by 45 cycles, each consisting of "95°C for 5 seconds and 54°C for the subsequent 30 seconds". The blood HBV DNA concentration was calculated as the average of two wells. Statistical calculations were performed under Two-tailed t-test conditions using Excel. As controls, GLS4 or a vehicle was administered instead of the compound CBT-209. FIG. 4 shows the obtained results.

As shown in FIG. 4, it was confirmed that, in the hepatitis B virus-infected human hepatocytes chimeric mice to which the compound CBT-209 was administered, the HBV DNA level was preferentially reduced compared with GLS4 or the vehicle.

### Inhibitory Effect on HBc T33N Mutation Resistant to Existing Capsid Formation Inhibitors

The HBV replication inhibitory activity of compounds was evaluated using the "HBV replication assay", an evaluation method described in WO 2018/30534 titled "Composition for treating hepatitis B, and method for evaluating replication activity of hepatitis B virus". Reporter pgRNA, HBc, HBV-pol, and HBx expression plasmids were introduced into HeLa cells by electroporation using NEPA21 manufactured by Nepa Gene Co., Ltd. As the HBc expression plasmids, in addition to the wild type (WT), HBc P25G and HBc T33N expression plasmids, in which mutations resistant to existing capsid inhibitors were introduced, were used. Cells were added to and cultured in plates to which serial dilutions of a compound had been previously added. After 24 hours of culture, the cells were treated with a hypotonic solution, cytoplasmic DNA was extracted, and HBV replication was measured by qPCR using a specific primer. The obtained HBV replication assay results (IC₅₀) are shown in Table 6, and WST-8 assay results (IC₂₅) are shown in Table 7.

**[Table 6]**

| | WT | HBc P25 | HBc T33N |
|---|---|---|---|
| CBT-209 | 0.051 | 0.433 | 8.961 |
| CBT-078 | 0.076 | 0.530 | 12.707 |
| GLS4 | 0.121 | 0.963 | 13.179 |

**[Table 7]**

| | WT | HBc P25 | HBc T33N |
|---|---|---|---|
| CBT-209 | > 20.000 | > 20.000 | > 20.000 |
| CBT-078 | > 20.000 | > 20.000 | > 20.000 |
| GLS4 | > 20.000 | > 20.000 | > 20.000 |

As shown in Table 6, it was confirmed that the compound CBT-209 showed stronger inhibitory activity than the conventional GLS4 and compound CBT-078. As shown in Table 7, it was also confirmed that the compound CBT-209 was nontoxic like the conventional GLS4 and compound CBT-078.

### Analysis of the Mechanism of Capsid Formation Inhibition by Electron Microscopy

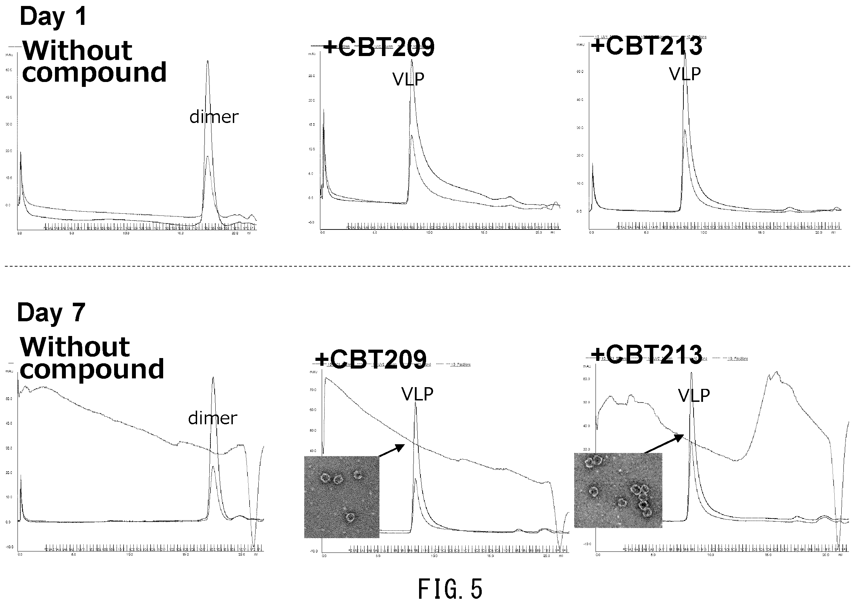

An expression plasmid in which the ubiquitin-like protein SUMO serving as a tag was fused to the sequence of the association domain (1-149) of HBc of genotype C was prepared and introduced into the *Escherichia coli* KRX strain, and a recombinant protein was expressed and purified. After the recombinant HBc (1-149) was cleaved with a SUMO protease to remove the tag, the dimer peak was separated by gel filtration and used for subsequent analyses. Eleven µM of HBc (1-149) and 15 µM of a CBT compound were mixed in a solution of 20 mM Tris-HCI (pH 7.5) and 100 mM NaCl, and after overnight incubation at 4°C, the association state was examined by gel filtration using Superose 6 10/30 GL. FIG. 5 shows the obtained results.

In the absence of the compound, HBc (1-149) was eluted at a position corresponding to the dimer, but when the compound CBT-209 or the compound CBT-213 was added, the elution peak shifted to the position corresponding to the capsid. This behavior is similar to that of the compound CBT-078. Furthermore, when the incubation time in the presence of the compound was extended to 7 days, an elution peak was observed only at the capsid position when the compound CBT-209 or the compound CBT-213 was added, as in the case of the overnight incubation. A fraction was collected from the elution peak at the position corresponding to the capsid, and a negatively stained image was captured using an electron microscope. When the compound CBT-209 was added or the compound CBT-213 was added, an image with a particle size close to that of an intact capsid formed with an increased HBc concentration or salt concentration without adding the compound was observed. In other words, the capsids formed by the compound CBT-209 and by the compound CBT-213 were stable even after 7 days of incubation.

FIG. 5 suggests that the compound CBT-209 and the compound CBT-213 bind to the HBc association interface and promote HBc association, thereby forming a hollow capsid in a stable state. In addition, it was confirmed that the compound CBT-209 and the compound CBT-213 also have antiviral activity even against viruses with the HBc T33N mutation, which is a mutation resistant to conventional capsid formation inhibitors.

## Claims

1. An anti-HBV agent comprising a compound of Formula (I) or a salt thereof: 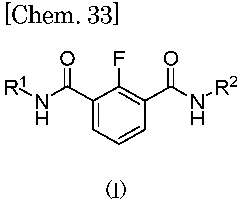 where
R¹ is a phenyl group substituted with a group represented by -CN or R³-NH-CO- and an alkyl group having 1 to 6 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 5; and
R² is a a phenyl group substituted with a halogen.

2. The anti-HBV agent according to claim 1, wherein the compound of Formula (I) is a compound of Formula (II): 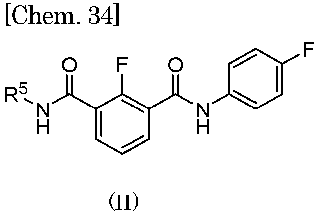 where
R⁵ is a phenyl group substituted with a group represented by R⁶-NH-CO- and an alkyl group having 1 to 6 carbon atoms,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 1 to 5.

3. A compound of Formula (I) or a salt thereof: 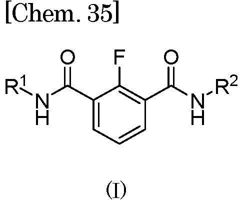 where
R¹ is a phenyl group substituted with a group represented by -CN or R³-NH-CO- and an alkyl group having 1 to 6 carbon atoms,
where R³ is an alkyl group having 1 to 10 carbon atoms or a group represented by the formula R⁴-(CH₂)ₙ₁-,
where R⁴ is CH₂=CH-, a phenyl group, a cyclohexyl group, or a morpholino group, and
n1 is an integer of 1 to 5; and
R² is a phenyl group substituted with a halogen.

4. A compound of Formula (II) or a salt thereof: 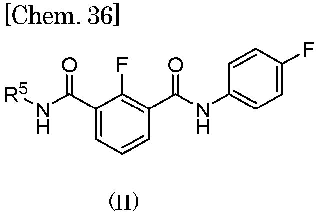 where
R⁵ is a phenyl group substituted with a group represented by R⁶-NH-CO- and an alkyl group having 1 to 6 carbon atoms,
where R⁶ is an alkyl group having 1 to 10 carbon atoms or a group represented by the formula R⁷-(CH₂)ₙ₂-,
where R⁷ is CH₂=CH-, a phenyl group, a cyclohexyl group, or a morpholino group, and
n2 is an integer of 1 to 5.

5. An HBV capsid formation inhibitor comprising the compound of Formula (I) or salt thereof according to claim 3 or the compound of Formula (II) or salt thereof according to claim 4.

6. A compound of Formula (III) or a salt thereof: 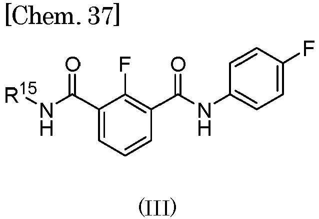 where
R¹⁵ is a phenyl group substituted with a group represented by R¹⁶-NH-CO- and an alkyl group having 1 to 6 carbon atoms,
where R¹⁶ is an alkyl group having 1 to 10 carbon atoms or a group represented by the formula R¹⁷-(CH₂)ₙ₃-,
where R¹⁷ is CH₂=CH-, a phenyl group, a cyclohexyl group, or a morpholino group, and
n3 is an integer of 1 to 5,
the compound of Formula (III) being labeled with ¹¹C, or labeled with ¹⁸F when the compound contains a fluorine atom.

7. A PET probe for imaging that targets a hepatitis B antigen protein, the PET probe comprising the compound of Formula (III) or salt thereof according to claim 6.
